# EUROPEAN PATENT APPLICATION

(11) **EP 4 302 768 A2**
(43) Date of publication of application: **10.01.2024**
(21) Application number: 23209126.4
(22) Date of filing: 22.06.2018
(51) Int. Cl.: A61K 35/17

(54) **METHODS FOR PRODUCING REGULATORY IMMUNE CELLS AND USES THEREOF**

(30) Priority: 22.06.2017 US 201762523637 P
(62) Divisional of application: 18820204.8
(71) Applicant: Board Of Regents, The University Of Texas System, Austin, TX 78701 (US)
(72) Inventor: REZVANI, Katy, Houston, 77030 (US); SHPALL, Elizabeth, Houston, 77030 (US); BASAR, Rafet, Houston, 77030 (US)
(74) Representative: J A Kemp LLP

(57) **Abstract**

Provided herein are methods for expanding populations of regulatory B cells comprising treating a population of B cells with IL-4 and CD40 ligand. Further provided herein are methods of expanding populations of regulatory T cells comprising expanding a population of T cells under Treg expansion conditions and selecting for CD9⁺ Tregs. Also provided herein are methods of treating immune disorders with the regulatory B cells and/or regulatory T cells.

## Description

The present application claims the priority benefit of United States Provisional Application Serial No. 62/523,637, filed June 22, 2017, the entire contents of which are hereby incorporated by reference.

### INCORPORATION OF SEQUENCE LISTING

The sequence listing that is contained in the file named "UTFCP1312WO.txt", which is 2 KB (as measured in Microsoft Windows) and was created on June 22, 2018, is filed herewith by electronic submission and is incorporated by reference herein.

### BACKGROUND

This invention was made with government support under Grant Nos. HHSH234200737001C and HHSH250201000011C awarded by the Health Resources and Services Administration. The government has certain rights in the invention.

### 1. Field

The present invention relates generally to the fields of medicine and immunology. More particularly, it concerns regulatory B cell and regulatory T cell production and uses thereof.

### 2. Description of Related Art

Regulatory T cells (Tregs) and regulatory B cells (Bregs) can suppress immune responses and play an important role against autoimmune diseases and provide transplantation tolerance. As peripheral blood only comprises a small percentage of these suppressive immune cells, *ex vivo* methods of expansion are used to generate sufficient numbers of the suppressive cells for *in vivo* treatment or prevention of immune-associated diseases. Current protocols for expansion of Tregs comprise TCR ligation and IL-2 (He *et al.,* 2016) while methods for expansion of Bregs include BCR ligation and CD40 ligand (Taitano *et al.,* 2016).

However, immune therapies, such as for inflammatory conditions, generally require a large number of cells and, thus, it is vital to optimize the methods of inducing *in vitro* T or B cell proliferation in order to maximize the number of Treg or Bregs produced and minimize the time required to produce the suppressive cells in sufficient numbers. Therefore, there is an unmet need for methods of efficient isolation and expansion methods to generate Tregs or Bregs for use in the treatment of various immune diseases including autoimmunity, infection, cancer, and cGVHD.

### SUMMARY

Accordingly, certain embodiments of the present disclosure provide methods and compositions concerning the expansion of Tregs or Bregs as well as methods for the use of these suppressive immune cells in the treatment and/or prevention of immune-mediated diseases.

In a first embodiment, there is provided an *in vitro* method of expanding Tregs comprising obtaining a population (*e*.*g*. isolated population) of CD4⁺ T cells; culturing the population of CD4⁺ T cells under Treg expansion conditions, thereby producing expanded Tregs; and selecting for CD9⁺ cells from the expanded Tregs, thereby obtaining a population of CD9⁺ Tregs. In some aspects, the CD4⁺ T cells are further defined as CD4⁺CD25⁺ T cells, such as CD4⁺CD25+CD9⁺ T cells.

In certain aspects, selecting is further defined as positive selection. In other aspects, selecting is further defined as negative selection. In some aspects, selecting comprises sorting for CD9⁺ Tregs. In particular aspects, sorting is further defined as antibody bead selection, fluorescence associated cell sorting (FACS), or magnetic-activated cell sorting (MACS).

In some aspects, Treg expansion conditions comprise culturing the CD4⁺ T cells in the presence of TCR ligation, IL-2, and an mTOR inhibitor. In specific aspects, TCR ligation comprises an anti-CD3 antibody and an anti-CD28 antibody. In certain aspects, the mTOR inhibitor is rapamycin. In some aspects, the Treg expansion conditions further comprise culturing the CD4⁺ T cells in the presence of a tumor necrosis factor receptor 2 (TNFR2) agonist, all-trans retinoic acid (ATRA), adenosine receptor (A2AR), and/or an A2AR agonist. In particular aspects, culturing is for 10-14 days, 8-10 days, 9-15 days, or 12-16 days, such as 8, 9, 10, 11, 12, 13, 14, 15, or 16 days.

In certain aspects, obtaining the population of CD4⁺ T cells comprises isolating T cells from stem cells, bone marrow, peripheral blood, or cord blood. In particular aspects, obtaining the population of CD4⁺ T cells comprises isolating the T cells from pooled cord blood, such as pooled cord blood. In some aspects, isolating comprises performing antibody bead selection or FACS.

In additional aspects, the CD4⁺ T cells or Tregs are engineered to have decreased or essentially no expression of glucocorticoid receptor. In some aspects, the CD4⁺ T cells or Tregs are engineered using one or more guide RNAs and a Cas9 enzyme.

In further aspects, the CD4⁺ T cells or Tregs are engineered to express a chimeric antigen receptor (CAR) and/or a T cell receptor (TCR).

In some aspects, the CD4⁺ T cells or Tregs are engineered to express a suicide gene. In certain aspects, the suicide gene is CD20, CD52, EGFRv3, or inducible caspase 9.

In a further embodiment, there is provided a population of CD9⁺ regulatory T cells produced according to the above embodiments and aspects or any of the methods described in the present disclosure.

In another embodiment, there is provided a pharmaceutical composition comprising the population of CD9⁺ regulatory T cells of the embodiments and a pharmaceutically acceptable carrier.

In yet another embodiment, there is provided an *in vitro* method of expanding suppressive regulatory B cells (Bregs) comprising obtaining a population (*e*.*g*., isolated population) of B cells; culturing the B cells in the presence of IL-4, CpG oligodeoxynucleotides (ODNs), and CD40 ligand (CD40L); and further expanding the B cells in the presence of IL-21, CD40L, and at least one inhibitor selected from the group consisting of a FOXO1 inhibitor, a mTOR inhibitor, and a STAT6 inhibitor, thereby producing suppressive Bregs.

In some aspects, the expansion culture may comprise one or more additional cytokines at one or more of the culture steps. In some aspects, the additional cytokine is IL-33.

In certain aspects, the culturing in the presence of IL-4, CpG, and CD40L further comprises the presence of a FOXO1 inhibitor, an mTOR inhibitor, and/or a STAT6 inhibitor.

In some aspects, the method further comprises washing the B cells prior to the expanding step. In certain aspects, the media may be replaced with fresh media, such as a half media replacement at any stage of the expansion. In some aspects, the media replacement is between the culturing and expanding steps, such as media comprising IL-4 and a signaling inhibitor.

In particular aspects, the CD40L is soluble CD40L (sCD40L). In some aspects, the FOXO1 inhibitor is AS1842856 or AS1708727. In particular aspects, the FOXO1 inhibitor is AS1842856. In some aspects, the mTOR inhibitor is torkinib, rapamycin, everolimus, temsirolimus, deforolimus, BGT226, SF1126, BEZ235, Gedatolisib, or SF1101. In particular aspects, the mTOR inhibitor is torkinib. In some aspects, the STAT6 inhibitor is AS1517499 or leflunomide. In particular aspects, the STAT6 inhibitor is AS1517499.

In additional aspects, the method further comprises contacting the Bregs with anti-miR-155. The Bregs may be contacted with anti-miR-155 at any stage of the expansion method. In some aspects, the Bregs are contacted with anti-miR-155 after the expansion in the presence of IL-21, CD40L, and at least one inhibitor.

In some aspects, obtaining the population of B cells comprises isolating B cells from a blood sample. In certain aspects, isolating comprises performing antibody bead selection or FACS. In some aspects, the blood sample is peripheral blood or cord blood. In some aspects, the cord blood is pooled from 2 or more individual cord blood units, such as from 3, 4, or 5 individual cord blood units. In some aspects, the population of B cells are CB mononuclear cells (CBMCs). In certain aspects, the population of B cells are CD5⁺CD1d^{hi} B cells. In particular aspects, the population of B cells are total B cells.

In certain aspects, the Bregs have the capacity to suppress the proliferation of (1)4 T cells (e.g., activated CD4⁺ T cells), such as suppression by at least 10%, 15%, 20%, 25%, 30%, 40%, 50%, 60%, 65%, 70%, 75%, 80%, 90%, 91%, 92%, 93%, 94%, or 95%. In particular aspects, the Bregs are human Bregs.

In some aspects, the culturing is for 1 to 5 days, such as 2, 3, or 4 days. In certain aspects, the expanding is for 5 to 10 days, such as 6, 7, 8, 9, or 10 days.

In a further embodiment, there is provided a population of regulatory B cells produced according to the methods of the embodiments described herein.

Also provided herein is a pharmaceutical composition comprising the population of regulatory B cells of the embodiments described herein and a pharmaceutically acceptable carrier.

In another embodiment, there is provided a method of treating an immune disorder in a subject comprising administering a therapeutically effective amount of the suppressive Tregs of the embodiments and/or the suppressive Bregs of the embodiments to the subject. In some aspects, the subject is administered suppressive Tregs. In some aspects, the subject is administered suppressive Tregs. In certain aspects, the the subject is administered suppressive Tregs and Bregs. In particular aspects, the subject is a human.

In certain aspects, the subject has been or is currently being administered a glucocorticoid therapy.

In some aspects, the Tregs and/or Bregs are allogeneic. In other aspects, the Tregs and/or Bregs are autologous.

In certain aspects, the subject has been previously been administered a cord blood transplantation (CBT). In some aspects, the Tregs and/or Bregs are administered concurrently with the CBT. In other aspects, the Tregs and/or Bregs are administered prior to or after the CBT.

In some aspects, the immune disorder is inflammation, graft versus host disease, transplant rejection, or an autoimmune disorder. In particular aspects, the immune disorder is graft versus host disease (GVHD). In specific aspects, the GVHD is chronic GVHD (cGVHD). In some aspects, the immune disorder is transplant rejection, and wherein the transplant is an organ transplant, bone marrow or other cell transplant, composite tissue transplant, or a skin graft. In certain aspects, the immune disorder is multiple sclerosis, inflammatory bowel disease, rheumatoid arthritis, type I diabetes, systemic lupus erythrematosus, contact hypersensitivity, asthma or Sjogren's syndrome.

In additional aspects, the method further comprises administering at least a second therapeutic agent. In some aspects, the at least a second therapeutic agent is a therapeutically effective amount of an immunomodulatory or an immunosuppressive agent. In certain aspects, the immunosuppressive agent is a calcineurin inhibitor, an mTOR inhibitor, an antibody, a chemotherapeutic agent irradiation, a chemokine, an interleukins or an inhibitor of a chemokine or an interleukin. In some aspects, Tregs, Bregs, and/or the at least a second therapeutic agent are administered intravenously, intraperitoneally, intratracheally, intratumorally, intramuscularly, endoscopically, intralesionally, percutaneously, subcutaneously, regionally, or by direct injection or perfusion.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present disclosure. The present disclosure may be better understood by reference to one or more of these drawings in combination with the detailed description of specific embodiments presented herein.

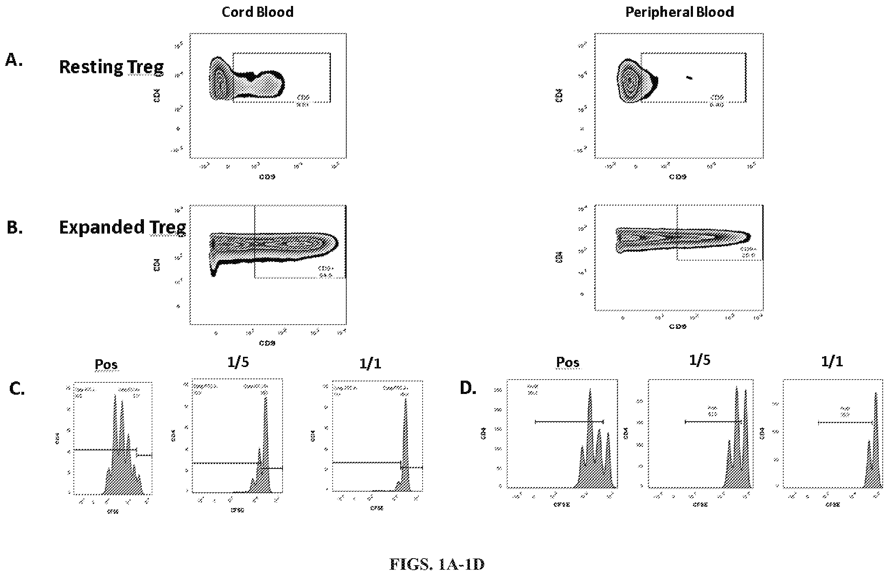

**FIGS. 1A-1D****:** CD9 is more highly expressed on cord blood T cells as compared to peripheral blood T cells. **(1A)** CD9 expression on resting CD4⁺ T cells isolated from cord blood. **(1B)** CD9 expression on expanded Tregs. **(1C-1D)** Suppressive activity of Tregs expanded from cord blood **(1C)** or peripheral blood **(1D).** 1:1 or 1:5 ratio indicates Treg/CD4⁺ T cells.

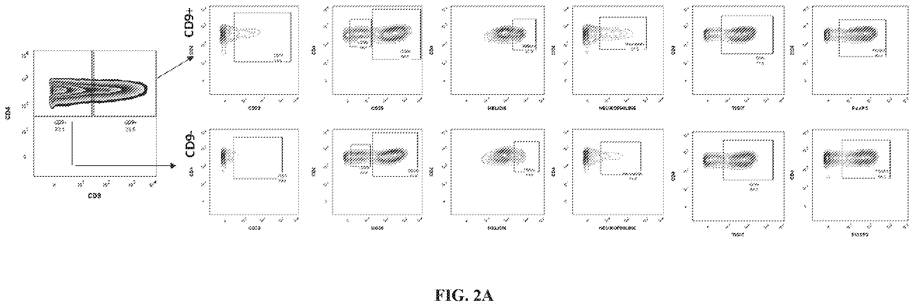

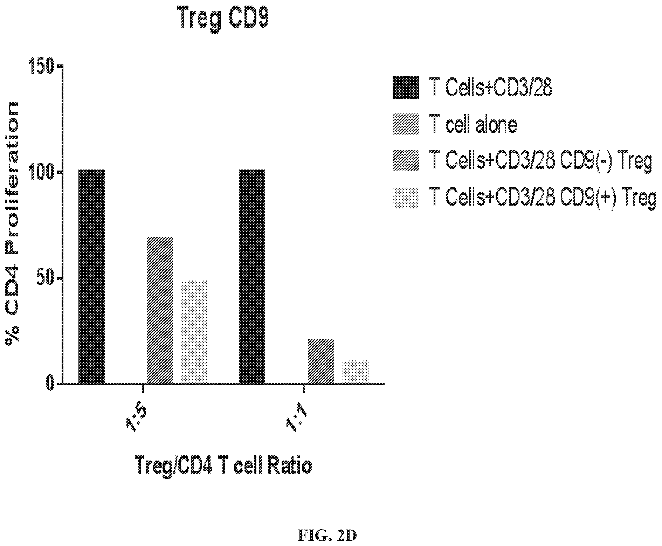

**FIGS. 2A-2D****: (2A)** CD9⁺ Tregs have higher expression of markers of suppressive activity include CD39, CD73, CD15s, Neurophiline, TIGIT, and FOXP3. **(2B)** Suppressive activity of CD9⁺ versus CD9⁻ Tregs expanded from peripheral blood. **(2C)** Suppressive activity of CD9⁺ versus CD9⁻ Tregs expanded from cord blood. **(2D)** CD9⁺ Tregs are more suppressive than CD9⁻ Tregs expanded from cord blood.

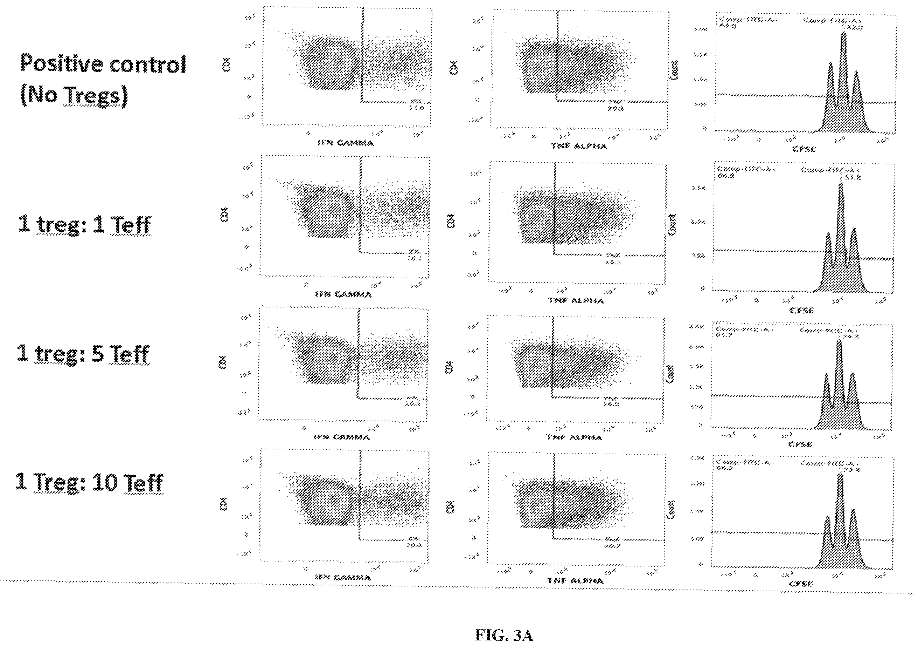

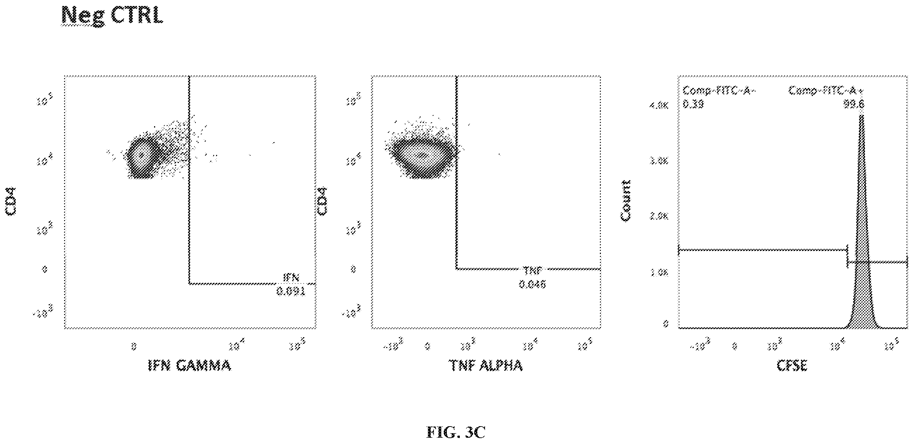

**FIGS. 3A-3C****: (3A)** Suppressive function of fresh cord blood Tregs generated by TCR ligation and IL-2. **(3B)** Suppressive function of fresh cord blood Tregs expanded with TCR ligation, IL-2 and rapamycin. **(3C)** Negative control of T cells prior to expansion.

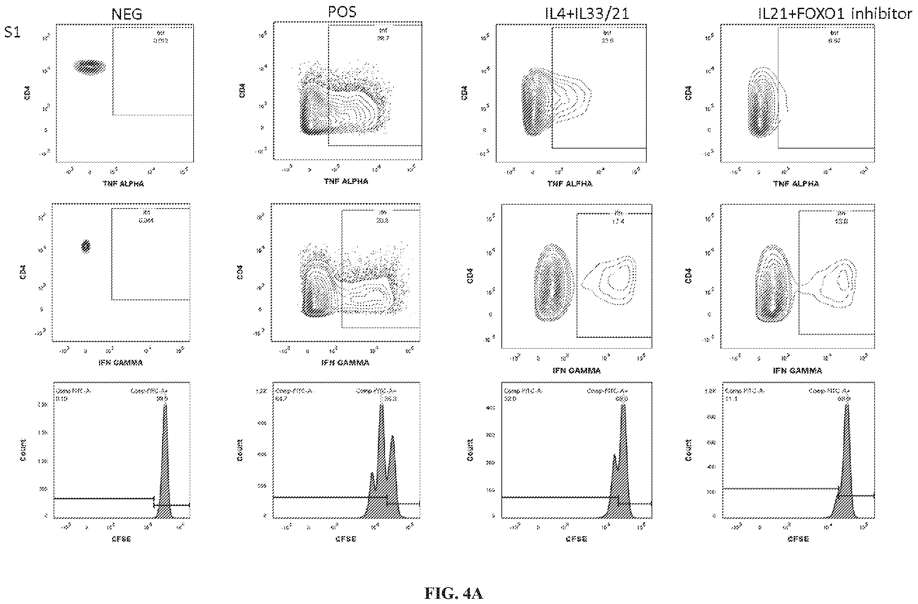

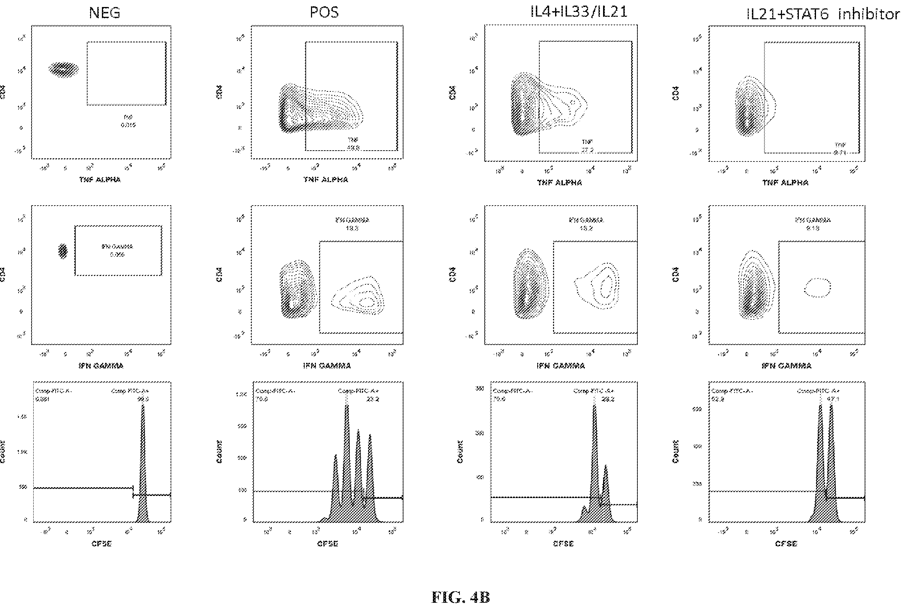

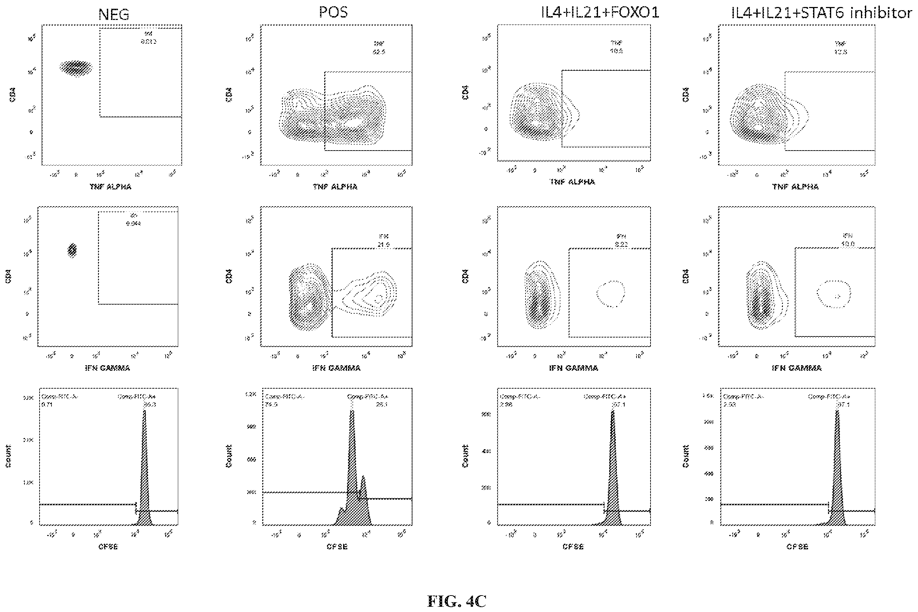

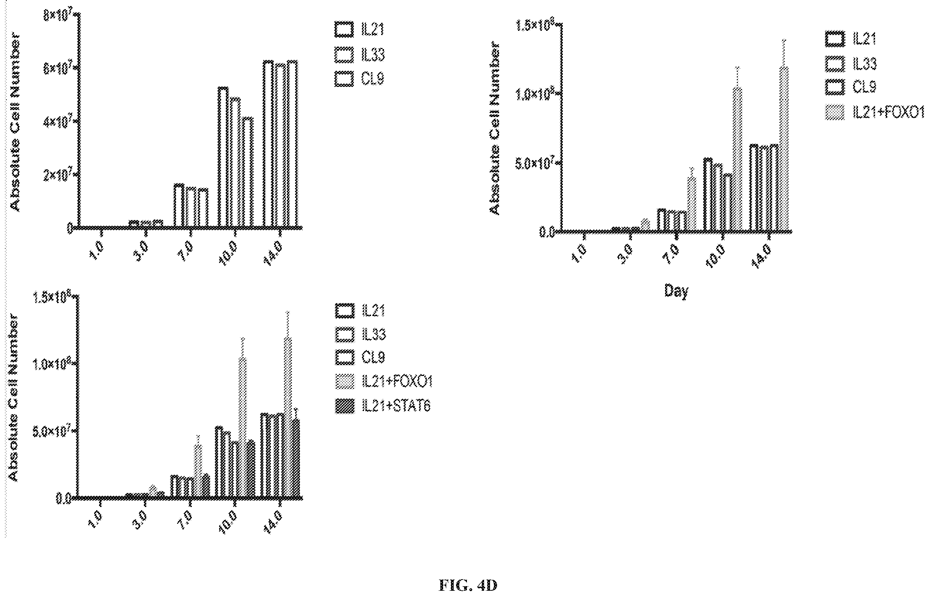

**FIGS. 4A-4D****: (4A)** Suppressive function of regulatory B cells expanded with IL-4+1L-33+IL-2 or IL-21+FOXO1 inhibitor as indicated. **(4B)** Suppressive function of Bregs expanded with IL21+STAT6 inhibitor. **(4C)** Suppressive function of Bregs expanded with IL-4+TL-21+FOXO1 inhibitor or IL-4+IL-21+STAT6 inhibitor. **(4D)** Cell number over 14 days of Breg expansion with indicated supplementation.

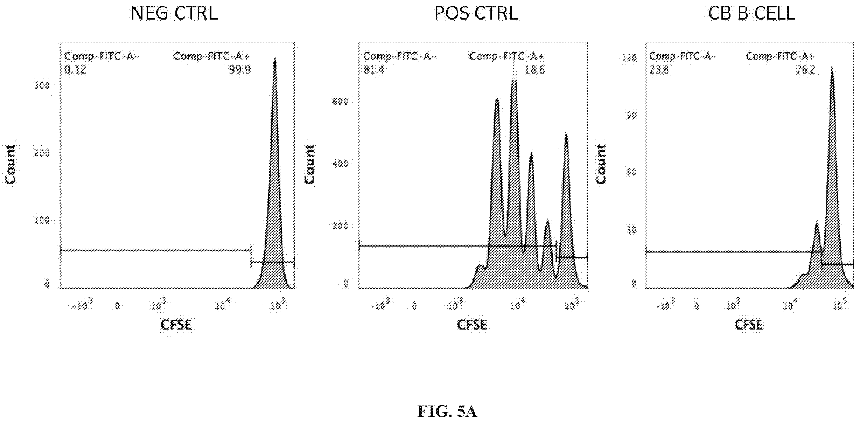

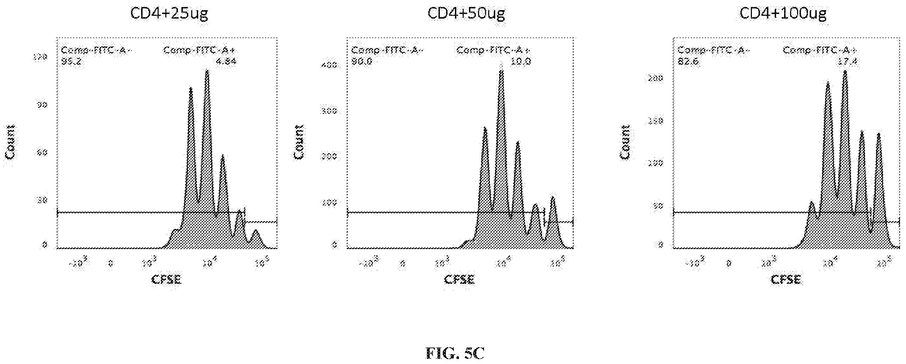

**FIGS. 5A-5C****: (5A)** CFSE assay of regulatory B cells expanded from cord blood. (5B) CFSE assay of CD4⁺ cord blood-derived B cells with 25, 50, or 100 µg of miRNA-155 inhibitor at different effector ratios. (5C) CD4⁺ B cells with 25, 50, or 100 µg of miRNA-155 inhibitor.

### DESCRIPTION OF ILLUSTRATIVE EMBODIMENTS

Certain embodiments of the present disclosure provide efficient methods of generating highly suppressive regulatory B cells (Bregs) and regulatory T cells (Tregs). Specifically, the immune cells produced by the present methods can be highly suppressive and of sufficient quantity for treatment of inflammatory conditions.

In one method, a starting population of T cells are isolated from a sample, such as peripheral blood, bone marrow, or cord blood. The T cells are expanded in the presence of TCR ligation and co-stimulation, IL-2, and an mTOR inhibitor, such as rapamycin. The TCR ligation and co-stimulation may comprise stimulation with anti-CD3 and anti-CD28 antibodies, such as antibodies on beads. Further, the Treg expansion may comprise TNFR2 agonist, ATRA or adenosine receptor agonist. Interestingly, the inventors have discovered a subset of Tregs defined phenotypically as CD4⁺CD25⁺CD9⁺ T cells that are highly suppressive. These T cells are present at higher frequencies in cord blood and may be selected for expansion to provide an even more suppressive Treg population. In certain aspects, the method for producing suppressive Tregs comprises selecting for CD9⁺ Tregs after expansion. Thus, the present methods may be significantly more efficient at producing large numbers of Tregs as compared to previous protocols.

Bregs may be expanded by culturing B cells (*e.g*., total B cells or a purified CD5⁺CD1d^{high} population of B cells) in the presence of CpG oligonucleotides, CD40 ligand (CD40L), and IL-4. The expansion culture may further comprise one or more signaling inhibitors, such as a FOXO1 inhibitor, mTOR inhibitor, and/or a STAT6 inhibitor. The starting population of B cells may be isolated from peripheral blood or cord blood. The initial expansion culture may be for about 3 to 10 days, such as 4 to 5 days. The Bregs may be further expanded, such as at day 5, in the presence of IL-21, CD40L and either IL-33 or a signaling inhibitor, such as a FOXO1 inhibitor, mTOR inhibitor, and/or a STAT6 inhibitor, such as for an additional 1 to 4 weeks, particularly 2 to 3 weeks. In addition, the inventors have found that exposure of B cells to anti-miR155 (*e.g.*, pharmacologic inhibition or genetic manipulation) further increases their suppressive function. Thus, in some embodiments, the B cells or expanded Bregs are contacted with anti-miR155 to further enhance their suppressive activity.

The Tregs or Bregs may be genetically modified (*e*.*g*., before expansion or after expansion) to silence the expression of glucocorticoid receptor (GR), such as by using RNA-guided endonuclease CRISPR and Cas9, to render them resistant to corticosteroids. The Treg or Bregs may be engineered to express a chimeric antigen receptor (CAR) and/or a T cell receptor (TCR). Further modifications may include a suicide switch that allows deletion of the cells to prevent any undue toxicity.

Accordingly, methods are also provided for harnessing this regulatory cell subsets for the manipulation of the immune and inflammatory responses, and for the treatment of immune-related diseases, disorders and conditions including inflammatory and autoimmune diseases, as well as immunosuppression and cancer in humans and other mammals. For example, these stimulated Bregs can be used to treat autoimmune or alloimmune disorders, such as graft versus host disorder (GVHD). Thus, the present disclosure provides compositions of expanded Tregs and Bregs which can be used for immunomodulation in a variety of immune-related disorders.

### I. Definitions

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

The term "cell" is herein used in its broadest sense in the art and refers to a living body that is a structural unit of tissue of a multicellular organism, is surrounded by a membrane structure that isolates it from the outside, has the capability of self-replicating, and has genetic information and a mechanism for expressing it. Cells used herein may be naturally-occurring cells or artificially modified cells (*e.g*., fusion cells or genetically modified cells).

The term "T cell" refers to T lymphocytes, and includes, but is not limited to, γδ⁺ T cells, NK T cells, CD4⁺ T cells and CD8⁺ T cells. CD4⁺ T cells include T_{H}0, T_{H}1 and T_{H}2 cells, as well as regulatory T cells (T_{reg}). There are at least three types of regulatory T cells: CD4⁺ CD25⁺ T_{reg}, CD25 T_{H}3 T_{reg}, and CD25 T_{R}l T_{reg}. "Cytotoxic T cell" refers to a T cell that can kill another cell. The majority of cytotoxic T cells are CD8+ MHC class I-restricted T cells, however some cytotoxic T cells are CD4⁺.

The term "B cell(s)" refers to a lymphocyte, a type of white blood cell (leukocyte) that expresses immunoglobulin on its surface and can ultimately develop into an antibody secreting a plasma cell. In one example, a B cell expresses CD19 (CD19⁺). An "immature B cell" is a cell that can develop into a mature B cell. Generally, pro-B cells (that express, for example, CD45 or B220) undergo immunoglobulin heavy chain rearrangement to become pro B pre B cells, and further undergo immunoglobulin light chain rearrangement to become an immature B cells. Immature B cells include T1 and T2 B cells.

A "regulatory B cell" (Breg) is a B cell that suppresses the immune response. Regulatory B cells can suppress T cell activation either directly or indirectly, and may also suppress antigen presenting cells, other innate immune cells, or other B cells. Regulatory B cells can be CD19⁺ or express a number of other B cell markers and/or belong to other B cell subsets. These cells can also secrete IL-10 which is enhanced by the stimulation methods provided herein.

A "B cell antigen receptor" or "BCR" refers to the B cell antigen receptor, which includes a membrane immunoglobulin antigen binding component, or a biologically active portion thereof (*i.e*, a portion capable of binding a ligand and/or capable of associating with a transducer component). The B cell receptor is generally composed of a surface bound IgM or IgD antibody associated with Ig-α and Ig-β heterodimers which are capable of signal transduction. The term "transmembrane domain of a B cell receptor" preferably refers to the transmembrane domain of the antibody part of the B cell receptor, *i.e*., the transmembrane domain of the IgM or IgD heavy chain. In some embodiments, the term "B cell receptor" or "BCR" preferably refers to a mature BCR and preferably excludes the pre-BCR which comprises a surrogate light chain.

A "CpG oligonucleotide" or "CpG oligodeoxynucleotides (ODN)" is an oligonucleotide which includes at least one unmethylated CpG dinucleotide. An oligonucleotide containing at least one unmethylated CpG dinucleotide is a nucleic acid molecule which contains an unmethylated cytosine-guanine dinucleotide sequence (*i.e.* "CpG DNA" or DNA containing a 5' cytosine followed by 3' guanosine and linked by a phosphate bond) and activates the immune system. The CpG oligonucleotides can be double-stranded or single-stranded. Generally, double-stranded molecules are more stable *in vivo,* while single-stranded molecules have increased immune activity.

The terms "nucleic acid" and "'oligonucleotide" are used interchangeably to mean multiple nucleotides (*i*.*e*. molecules comprising a sugar *(e.g.* ribose or deoxyribose) linked to a phosphate group and to an exchangeable organic base, which is either a substituted pyrimidine *(e.g.* cytosine (C), thymine (T) or uracil (U)) or a substituted purine *(e.g.* adenine (A) or guanine (G)). As used herein, the terms refer to oligoribonucleotides as well as oligodeoxyribonucleotides. The terms shall also include polynucleosides (*i.e.* a polynucleotide minus the phosphate) and any other organic base containing polymer. Nucleic acid molecules can be obtained from existing nucleic acid sources (*e*.*g*. genomic or cDNA)_ but are preferably synthetic (*e*.*g*. produced by oligonucleotide synthesis).

As used herein, the term "antigen" is a molecule capable of being bound by an antibody or T-cell receptor. An antigen may generally be used to induce a humoral immune response and/or a cellular immune response leading to the production of B and/or T lymphocytes.

An "immune disorder," "immune-related disorder," or "immune-mediated disorder" refers to a disorder in which the immune response plays a key role in the development or progression of the disease. Immune-mediated disorders include autoimmune disorders, allograft rejection, graft versus host disease and inflammatory and allergic conditions.

An "immune response" is a response of a cell of the immune system, such as a B cell, or a T cell, or innate immune cell to a stimulus. In one embodiment, the response is specific for a particular antigen (an "antigen-specific response").

An "epitope" is the site on an antigen recognized by an antibody as determined by the specificity of the amino acid sequence. Two antibodies are said to bind to the same epitope if each competitively inhibits (blocks) binding of the other to the antigen as measured in a competitive binding assay. Alternatively, two antibodies have the same epitope if most amino acid mutations in the antigen that reduce or eliminate binding of one antibody reduce or eliminate binding of the other. Two antibodies are said to have overlapping epitopes if each partially inhibits binding of the other to the antigen, and/or if some amino acid mutations that reduce or eliminate binding of one antibody reduce or eliminate binding of the other.

An "autoimmune disease" refers to a disease in which the immune system produces an immune response (for example, a B-cell or a T-cell response) against an antigen that is part of the normal host (that is, an autoantigen), with consequent injury to tissues. An autoantigen may be derived from a host cell, or may be derived from a commensal organism such as the micro-organisms (known as commensal organisms) that normally colonize mucosal surfaces.

The term "Graft-Versus-Host Disease (GVHD)" refers to a common and serious complication of bone marrow or other tissue transplantation wherein there is a reaction of donated immunologically competent lymphocytes against a transplant recipient's own tissue. GVHD is a possible complication of any transplant that uses or contains stem cells from either a related or an unrelated donor. In some embodiments, the GVHD is chronic GVHD (cGVHD).

A "parameter of an immune response" is any particular measurable aspect of an immune response, including, but not limited to, cytokine secretion (IL-6, IL-10, IFN-y, etc.), chemokine secretion, altered migration or cell accumulation, immunoglobulin production, dendritic cell maturation, regulatory activity, number of regulatory B cells and proliferation of any cell of the immune system. Another parameter of an immune response is structural damage or functional deterioration of any organ resulting from immunological attack. One of skill in the art can readily determine an increase in any one of these parameters, using known laboratory assays. In one specific non-limiting example, to assess cell proliferation, incorporation of ³H-thymidine can be assessed. A "substantial" increase in a parameter of the immune response is a significant increase in this parameter as compared to a control. Specific, non-limiting examples of a substantial increase are at least about a 50% increase, at least about a 75% increase, at least about a 90% increase, at least about a 100% increase, at least about a 200% increase, at least about a 300% increase, and at least about a 500% increase. Similarly, an inhibition or decrease in a parameter of the immune response is a significant decrease in this parameter as compared to a control. Specific, non-limiting examples of a substantial decrease are at least about a 50% decrease, at least about a 75% decrease, at least about a 90% decrease, at least about a 100% decrease, at least about a 200% decrease, at least about a 300% decrease, and at least about a 500% decrease. A statistical test, such as a non-parametric ANOVA, or a T-test, can be used to compare differences in the magnitude of the response induced by one agent as compared to the percent of samples that respond using a second agent. In some examples, p^0.05 is significant, and indicates that the chance that an increase or decrease in any observed parameter is due to random variation is less than 5%. One of skill in the art can readily identify other statistical assays of use.

"Treating" or treatment of a disease or condition refers to executing a protocol, which may include administering one or more drugs to a patient, in an effort to alleviate signs or symptoms of the disease. Desirable effects of treatment include decreasing the rate of disease progression, ameliorating or palliating the disease state, and remission or improved prognosis. Alleviation can occur prior to signs or symptoms of the disease or condition appearing, as well as after their appearance. Thus, "treating" or "treatment" may include "preventing" or "prevention" of disease or undesirable condition. In addition, "treating" or "treatment" does not require complete alleviation of signs or symptoms, does not require a cure, and specifically includes protocols that have only a marginal effect on the patient.

The term "therapeutic benefit" or "therapeutically effective" as used throughout this application refers to anything that promotes or enhances the well-being of the subject with respect to the medical treatment of this condition. This includes, but is not limited to, a reduction in the frequency or severity of the signs or symptoms of a disease. For example, treatment of cancer may involve, for example, a reduction in the size of a tumor, a reduction in the invasiveness of a tumor, reduction in the growth rate of the cancer, or prevention of metastasis. Treatment of cancer may also refer to prolonging survival of a subject with cancer.

"Subject" and "patient" refer to either a human or non-human, such as primates, mammals, and vertebrates. In particular embodiments, the subject is a human.

The term "antibody" herein is used in the broadest sense and specifically covers monoclonal antibodies (including full length monoclonal antibodies), polyclonal antibodies, multispecific antibodies (*e*.*g*., bispecific antibodies), and antibody fragments so long as they exhibit the desired biological activity.

The term "monoclonal antibody" as used herein refers to an antibody obtained from a population of substantially homogeneous antibodies, *e*.*g*., the individual antibodies comprising the population are identical except for possible mutations, *e.g*., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, *etc.,* and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The phrases "pharmaceutical or pharmacologically acceptable" refers to molecular entities and compositions that do not produce an adverse, allergic, or other untoward reaction when administered to an animal, such as a human, as appropriate. The preparation of a pharmaceutical composition comprising an antibody or additional active ingredient will be known to those of skill in the art in light of the present disclosure. Moreover, for animal (*e*.*g*., human) administration, it will be understood that preparations should meet sterility, pyrogenicity, general safety, and purity standards as required by FDA Office of Biological Standards.

As used herein, "pharmaceutically acceptable carrier" includes any and all aqueous solvents (*e*.*g*., water, alcoholic/aqueous solutions, saline solutions, parenteral vehicles, such as sodium chloride, Ringer's dextrose, etc.), non-aqueous solvents (*e*.*g*., propylene glycol, polyethylene glycol, vegetable oil, and injectable organic esters, such as ethyloleate), dispersion media, coatings, surfactants, antioxidants, preservatives (*e*.*g*., antibacterial or antifungal agents, anti-oxidants, chelating agents, and inert gases), isotonic agents, absorption delaying agents, salts, drugs, drug stabilizers, gels, binders, excipients, disintegration agents, lubricants, sweetening agents, flavoring agents, dyes, fluid and nutrient replenishers, such like materials and combinations thereof, as would be known to one of ordinary skill in the art. The pH and exact concentration of the various components in a pharmaceutical composition are adjusted according to well-known parameters.

The term "haplotyping or tissue typing" refers to a method used to identify the haplotype or tissue types of a subject, for example by determining which HLA locus (or loci) is expressed on the lymphocytes of a particular subject. The HLA genes are located in the major histocompatibility complex (MHC), a region on the short arm of chromosome 6, and are involved in cell-cell interaction, immune response, organ transplantation, development of cancer, and susceptibility to disease. There are six genetic loci important in transplantation, designated HLA-A, HLA-B, HLA-C, and HLA-DR, HLA-DP and HLA-DQ. At each locus, there can be any of several different alleles.

A widely used method for haplotyping uses the polymerase chain reaction (PCR) to compare the DNA of the subject, with known segments of the genes encoding MHC antigens. The variability of these regions of the genes determines the tissue type or haplotype of the subject. Serologic methods are also used to detect serologically defined antigens on the surfaces of cells. HLA-A, -B, and -C determinants can be measured by known serologic techniques. Briefly, lymphocytes from the subject (isolated from fresh peripheral blood) are incubated with antisera that recognize all known HLA antigens. The cells are spread in a tray with microscopic wells containing various kinds of antisera. The cells are incubated for 30 minutes, followed by an additional 60-minute complement incubation. If the lymphocytes have on their surfaces antigens recognized by the antibodies in the antiserum, the lymphocytes are lysed. A dye can be added to show changes in the permeability of the cell membrane and cell death. The pattern of cells destroyed by lysis indicates the degree of histologic incompatibility. If, for example, the lymphocytes from a person being tested for HLA-A3 are destroyed in a well containing antisera for HLA-A3, the test is positive for this antigen group.

"Interferon-gamma (IFN-γ)" refers to a protein produced by T lymphocytes in response to specific antigen or mitogenic stimulation. The term includes naturally occurring IFN-γ peptides and nucleic acid molecules and IFN-γ fragments and variants that retain full or partial IFN-γ biological activity. Sequences for IFN-γ are publicly available (for example, exemplary IFN-γ mRNA sequences are available from GenBank Accession Nos: BC070256; AF506749; and J00219, and exemplary IFN-γ protein sequences are available from GenBank Accession Nos: CAA00226; AAA72254; and 0809316A).

"Interleukin (IL)-2" refers to a growth factor for all subpopulations of T-lymphocytes. It is an antigen-unspecific proliferation factor for T-cells that induces cell cycle progression in resting cells, and allows clonal expansion of activated T-lymphocytes. The term includes naturally occurring IL-2 peptides and nucleic acid molecules and IL-2 fragments and variants that retain full or partial IL-2 biological activity. Sequences for IL-2 are publicly available (for example, exemplary IL-2 mRNA sequences are available from GenBank Accession Nos: BC066254; BC066257; E00978; and NM_053836, and exemplary DL-2 protein sequences are available from GenBank Accession Nos: AAD14263; AAG53575; and AAK52904).

The term "culturing" refers to the *in vitro* maintenance, differentiation, and/or propagation of cells in suitable media. By "enriched" is meant a composition comprising cells present in a greater percentage of total cells than is found in the tissues where they are present in an organism.

An "isolated" biological component (such as a portion of hematological material, such as blood components) refers to a component that has been substantially separated or purified away from other biological components of the organism in which the component naturally occurs. An isolated cell is one which has been substantially separated or purified away from other biological components of the organism in which the cell naturally occurs.

As used herein, the term "substantially" is used to represent a composition comprising at least 80% of the desired component, more preferably 90% of the desired component, or most preferably 95% of the desired component. In some embodiments, the composition comprises at least 80%, 82%, 84%, 86%, 88%, 90%, 91%, 92%, 93%, 94%, 95%, 96%, 97%, or 98% of the desired component.

### II. Regulatory B Cells

Some embodiments of the present disclosure concern the isolation and expansion of regulatory B cells (Breg). Accordingly, populations of highly suppressive Bregs are disclosed herein. The Bregs can be characterized by the capacity to inhibit the proliferation of effector T cells, such as CD4⁺ T cells, inhibit the production of inflammatory cytokines (*e*.*g*., IFNy and TNFα) by effector T cells, and to produce interleukin-10 (IL-10).

### A. Isolation of Population of B Cells

The isolated population of B cells may be obtained from subjects, particularly human subjects. The B cells can be obtained from a subject of interest, such as a subject suspected of having a particular disease or condition, a subject suspected of having a predisposition to a particular disease or condition, or a subject who is undergoing therapy for a particular disease or condition. B cells can be collected from any location in which they reside in the subject including, but not limited to, blood, cord blood, spleen, thymus, lymph nodes, and bone marrow. The isolated B cells may be analyzed directly, or they can be stored until the assay is performed, such as by freezing.

In particular embodiments, the B cells are isolated from blood, such as peripheral blood, bone marrow or cord blood, or derived from stem cells or iPSCs. In some aspects, B cells isolated from cord blood have enhanced immunomodulation capacity, such as measured by CD4- or CD8-positive T cell suppression. In specific aspects, the B cells are isolated from pooled blood, particularly pooled cord blood, for enhanced immunomodulation capacity. The pooled blood may be from 2 or more sources, such as 3, 4, 5, 6, 7, 8, 9, 10 or more sources (e.g., donor subjects).

The population of B cells can be obtained from a subject in need of therapy or suffering from a disease associated with reduced regulatory B cell activity. Thus, the cells will be autologous to the subject in need of therapy. Alternatively, the population of B cells can be obtained from a donor, preferably a histocompatibility matched donor.

When the population of regulatory B cells is obtained from a donor distinct from the subject, the donor is preferably allogeneic, provided the cells obtained are subject-compatible in that they can be introduced into the subject. Allogeneic donor cells may or may not be human-leukocyte-antigen (HLA)-compatible. To be rendered subject-compatible, allogeneic cells can be treated to reduce immunogenicity (Fast *et al.,* 2004).

Methods for the isolation and quantitation of populations of cells are well known in the art, and the isolation and quantitation of regulatory B cells, such as CD19⁺ cells can be accomplished by any means known to one of skill in the art. Magnetic beads directed against CD19 or fluorescence activated cell sorting (FACS), or other cell isolation methods, can be used to isolate cells that are CD19⁺, and particularly that also produce IL-10. Regulatory B cells can also be isolated that express CD 19 and are CD38^{hi}CD24^{hi}, IgM^{hi}IgD⁺CD10⁺CD27⁻, CD38^{int}CD24^{int} or IgM^{int}IgD⁺CD10⁻CD27⁻ or that belong to any other B cell subpopulation. In particular aspects, the B cells are CD5⁺CD1d^{hi} B cells. In one embodiment, labeled antibodies specifically directed to one or more cell surface markers are used to identify and quantify regulatory B cells, such as CD19⁺ cells. The antibodies can be conjugated to other compounds including, but not limited to, enzymes, magnetic beads, colloidal magnetic beads, haptens, fluorochromes, metal compounds, radioactive compounds or drugs. The enzymes that can be conjugated to the antibodies include, but are not limited to, alkaline phosphatase, peroxidase, urease and B-galactosidase. The fluorochromes that can be conjugated to the antibodies include, but are not limited to, fluorescein isothiocyanate, tetramethylrhodamine isothiocyanate, phycoerythrin, allophycocyanins and Texas Red.

Regulatory B cells can be enriched by selecting cells having the CD19⁺ surface marker and separating using automated cell sorting such as fluorescence-activated cell sorting (FACS). To enhance enrichment, positive selection may be combined with negative selection; *i.e*., by removing cells having surface markers specific to non-B cells and/or those specific to non-regulatory B cells. Exemplary surface markers specific to non-regulatory B cells include CD3, CD4, CD7, CD8, CD15, CD16, CD34, CD56, CD57, CD64, CD94, CD116, CD134, CD157, CD163, CD208, F4/80, Gr-1, and TCR.

In some examples, regulatory B cells, such as CD19⁺ cells, are isolated by contacting the cells with an appropriately labeled antibody to identify the cells of interest followed by a separation technique such as FACs or antibody-binding beads. However, other techniques of differing efficacy may be employed to purify and isolate desired populations of cells. The separation techniques employed should maximize the retention of viability of the fraction of the cells to be collected. The particular technique employed will, of course, depend upon the efficiency of separation, cytotoxicity of the method, the case and speed of separation, and what equipment and/or technical skill is required.

Additional separation procedures may include magnetic separation, using antibody-coated magnetic beads, affinity chromatography, cytotoxic agents, either joined to a monoclonal antibody or used in conjunction with complement, and "panning," which utilizes a monoclonal antibody attached to a solid matrix, or another convenient technique. Antibodies attached to magnetic beads and other solid matrices, such as agarose beads, polystyrene beads, hollow fiber membranes and plastic Petri dishes, allow for direct separation. Cells that are bound by the antibody can be removed from the cell suspension by simply physically separating the solid support from the cell suspension. The exact conditions and duration of incubation of the cells with the solid phase-linked antibodies will depend upon several factors specific to the system employed. The selection of appropriate conditions, however, is well known in the art.

Unbound cells then can be eluted or washed away with physiologic buffer after sufficient time has been allowed for the cells expressing a marker of interest (for example, CD19⁺) to bind to the solid-phase linked antibodies. The bound cells are then separated from the solid phase by any appropriate method, depending mainly upon the nature of the solid phase and the antibody employed, and quantified using methods well known in the art. In one specific, non-limiting example, bound cells separated from the solid phase are quantified by flow cytometry.

Regulatory B cells, such as CD19⁺ B cells, can also be isolated by negatively selecting against cells that are not regulatory B cells. This can be accomplished by performing a lineage depletion, wherein cells are labeled with antibodies for particular lineages such as the T lineage, the macrophage/monocyte lineage, the dendritic cell lineage, the granulocyte lineages, the erythrocytes lineages, the megakaryocytes lineages, and the like. Cells labeled with one or more lineage specific antibodies can then be removed either by affinity column processing (where the lineage marker positive cells are retained on the column), by affinity magnetic beads or particles (where the lineage marker positive cells are attracted to the separating magnet), by "panning" (where the lineage marker positive cells remain attached to the secondary antibody coated surface), or by complement-mediated lysis (where the lineage marker positive cells are lysed in the presence of complement by virtue of die antibodies bound to their cell surface). Another lineage depletion strategy involves tetrameric complex formation. Cells are isolated using tetrameric anti-human antibody complexes (for example, complexes specific for multiple markers on multiple cell types that are not markers of regulatory B cells, and magnetic colloid in conjunction with STEMSTEP^{™} columns (Stem Cell Technologies, Vancouver, Canada). The cells can then optionally be subjected to centrifugation to separate cells having tetrameric complexes bound thereto from all other cells.

In a certain embodiment, the isolated B cells from a single donor or pooled donors can be stored for a future use. In this regard, the isolated B cell population can be cryopreserved. Cryopreservation is a process where cells or whole tissues are preserved by cooling to low sub-zero temperatures, such as 77 K or -196° C. in the presence of a cryoprotectant. Storage by cryopreservation includes, but is not limited to, storage in liquid nitrogen, storage in freezers maintained at a constant temperature of about 0° C., storage in freezers maintained at a constant temperature of about -20° C., storage in freezers maintained at a constant temperature of about -80° C., and storage in freezers maintained at a constant temperature of lower than about -80° C. In one aspect of this embodiment, the cells may be "flash-frozen," such as by using in ethanol/dry ice or in liquid nitrogen prior to storage. In another aspect of this embodiment, the cells can be preserved in medium comprising a cryoprotectant including, but not limited to dimethyl sulfoxide (DMSO), glycerol, ethylene glycol, propylene glycol, sucrose, and trehalose. Other methods of storing biological matter are well known to those of skill in the art, see for example U.S. Patent Publication No. 2007/0078113, incorporated by reference herein.

### B. Expansion of Regulatory B Cells

The isolated populated of B cells may then be expanded to increase the number of cells and/or to increase the suppressive capacity of the regulatory B cells. Expansion of the regulatory B cell population can be achieved by contacting the population of regulatory B cells with stimulatory composition sufficient to cause an increase in the number of regulatory B cells. This may be accomplished by contacting the isolated CD19⁺ B cells with a mitogen, cytokine, growth factor, or antibody, such as an antibody that specifically binds to the B cell receptor or feeder cells. The regulatory B cells can be expanded at least 2-fold, 5-fold, 10-fold, such as at least 50, 100, 200, 300, 500, 800, 1000, 10,000, or 100,000-fold.

The present disclosure provides methods for the expansion of the isolated B cells by treating the cells with one or more expansion agents to enhance their suppressive capacity. The expanded regulatory B cell population can include at least 5%, at least 10%, at least 20%, at least 30%, at least 40%, at least 50%, at least 60%, at least 90%, at least 91%, at least 92%, at least 93%, at least 94%, at least 95%, at least 99%, or 100% regulatory B cells that produce IL-10 or exert their suppressive function through other mechanisms.

The expansion agents may include CD40 agonist, such as CD40 ligand (CD40L), particularly soluble CD40 and CpG nucleotides. Further expansion agents can include cytokines such as IL-4, IL-21, IL-33 or a combination thereof. The culture may also comprise IL-2.

In some aspects, the isolated population of B cells are first cultured in the presence of IL-4, CpG, and CD40L, such as for about 3-4 days. The media may further comprise one or more signaling inhibitors. For example, the B cells may be cultured in the presence of IL-4, CpG, CD40L, and a signaling inhibitor, such as a FOXO1 inhibitor, a STAT6 inhibitor, and/or an mTOR inhibitor. During the first expansion step, the media of the B cell culture may be replaced with fresh media. The fresh media may comprise one or more of IL-4, CpG, CD40L, and a signaling inhibitor. In some aspects, the fresh media may comprise IL-4 and a signaling inhibitor. Next, the B cells are then washed and further cultured in the presence of IL-21 in combination with a signaling inhibitor, such as a FOXO1 inhibitor, a STAT6 inhibitor, and/or an mTOR inhibitor. This second step of expansion may comprise CD40L in the presence or absence of CpG.

The expansion culture may further comprise one or more additional cytokines, such as IL-33. For example, the second culturing step may comprise IL-21, CD40L, and IL33. In another example, the second culture step may comprise IL-21, CD40L, and a FOXO1 inhibitor. The total expansion culture may be performed for about 8-15 days, such as 10-14 days.

The suppressive function of the Bregs may be enhanced by the addition of anti-miR-155. The anti-miR-155 may be added to the expansion culture at any step of the process. However, in particular aspects, the anti-miR-155 is added to the Breg culture after expansion to further enhance the suppressive activity of the Bregs. For example, the anti-miR-155 may be added to the culture of Bregs at day 12-20 of expansion culture, such as day 13, 14, 15, or 16.

### 1. CD40 Ligand

In certain embodiments, the isolated B cells are cultured with a CD40 agonist, such as soluble CD40L, alone or in combination with other expansion agents. The term "CD40" refers to any, preferably naturally occurring, CD40 protein. CD40 is a transmembrane glycoprotein cell surface receptor that shares sequence homology with the tumor necrosis factor α (TNF-a) receptor family and was initially identified as a B cell surface molecule that induced B cell growth upon ligation with monoclonal antibodies.

Its ligand CD40L, also termed CD 154, is a 34-39 kDa type II integral membrane protein belonging to the TNF gene superfamily and is mainly expressed on activated T cells. Engagement of CD40 by its ligand leads to trimeric clustering of CD40 and the recruitment of adaptor proteins known as TNF receptor-associated factors (TRAFs) to the cytoplasmic tail of CD40. CD40L, also known as CD154, TNFSF5, TRAP, and gp39, is a member of the TNF superfamily which may trimerize to bind and activate CD40, as well as alpha Ilb-beta3 integrin. CD40L is about 30-kDa, the full-length version has 261 amino acids of which the Extra Cellular Domain (ECD) is amino acids 45-261). It is a type II membrane glycoprotein. In some physiological contexts, CD40L is processed to yield a soluble form comprised of amino acids 113-261.

As used herein, the term "CD40-L" includes soluble CD40-L polypeptides lacking transmembrane and intracellular regions, mammalian homologs of human CD40-L, analogs of human or murine CD40-L or derivatives of human or murine CD40-L. A CD40-L analog, as referred to herein, is a polypeptide substantially homologous to a sequence of human or murine CD40-L but which has an amino acid sequence different from native sequence CD40-L (human or murine species) polypeptide because of one or a plurality of deletions, insertions or substitutions. Analogs of CD40-L can be synthesized from DNA constructs prepared by oligonucleotide synthesis and ligation or by site-specific mutagenesis techniques.

In some embodiments, one or more CD40 agonists, such as CD40 ligands and/or agonistic anti-CD40 antibodies, may be used in combination with one or more other expansion agents to enhance expansion of Bregs. For example, the CD40 agonist is an agonistic anti-CD40 antibody, or antigen-binding fragment thereof, including, but not limited to, at least a first scFv, Fv, Fab', Fab or F(ab')₂ antigen-binding region of an anti-CD40 antibody. In certain aspects, the CD40 agonist is a human, humanized or part- human chimeric anti-CD40 antibody or antigen-binding fragment thereof. In other aspects, the CD40 agonist is an anti-CD40 monoclonal antibody, including, but not limited to, the G28-5, mAb89, EA-5 or S2C6 monoclonal antibody, or an antigen-binding fragment thereof.

In particular embodiments, the CD40 agonist is soluble CD40L, (sCD40L). Soluble CD40-L comprises an extracellular region of CD40-L or a fragment thereof. For example, soluble monomeric CD40L is described in U.S. Patent No. 6,264,951 and variants are described in International Publication No. WO2005/035570. CD40-L may also be obtained by mutations of nucleotide sequences coding for a CD40-L polypeptide. The B cells may be contacted with soluble CD40L, at a concentration of about 10 to 500 ng/mL, such as about 20 to 200 ng/mL, such as about 30 to 150 ng/mL, such as about 50, 75, 80, 90, 95, 100, 110, or 120 ng/mL, particularly about 100 ng/mL. In some aspects, the B cells are cultured in the presence of CD40L at a concentration of 100-500 ng/mL, such as 150, 200, 250, 300, 350, 400, or 450 ng/mL.

### 2. Cytokines

The expansion of the isolated B cells to highly suppressive Bregs may also comprise contacting the B cells with one or more cytokines, such as, but not limited to, IL-4, IL-21, IL-33, TL-2, IL-7, IL-10, IL-21, IL-35, and BAFF. In some aspects, the B cells are first contacted with IL-4 and then cultured in the presence of IL-21. The cytokines may be present at a concentration of about 10 to 500 lU/mL, such as about 50 to 200 IU/mL, such as about 75 to 150 TU/mL, particularly about 100 TU/mL. In some aspects, the IL-4 is present at a concentration of about 0.1 to 10 ng/mL in the expansion culture, such as about 1 to 5 ng/mL, for example 2, 2.5, 3, 3.5, 4, or 4.5 ng/mL or any range derivable therein. In specific aspects, the IL-21 is present in the expansion culture at a concentration of about 10 to 250 ng/mL, such as 25-50, 50-75, 75-125, 125-150, 150-175, 175-225, or 225-250 ng/mL.

### 3. mTOR Inhibitor

mTOR inhibitors are a class of drugs that inhibit the mechanistic target of rapamycin (mTOR), which is a serine/threonine-specific protein kinase that belongs to the family of phosphatidylinositol-3 kinase (PI3K) related kinases (PIKKs). Exemplary mTOR inhibitors that may be used in the present methods include, but are not limited to, rapamycin, everolimus, temsirolimus, deforolimus, BGT226, SF1126, BEZ235, Gedatolisib, and SF1101. In some aspects, the mTOR inhibitor is PP42 (also known as torkinib). The mTOR inhibitor may be present at a concentration of about 50 to 500 nM, such as 100 to 400 nm, particularly 150, 200, 250, 300, or 350 nM.

### 4. FOXO1 Inhibitor

Forkhead box protein O1 (FOXO1) also known as forkhead in rhabdomyosarcoma is a protein that in humans is encoded by the FOXO1 gene. FOXO1 is a transcription factor that plays important roles in regulation of gluconeogenesis and glycogenolysis by insulin signaling, and is also central to the decision for a preadipocyte to commit to adipogenesis. Exemplary FOXO1 inhibitors that may be used in the present methods include, but are not limited to, AS1842856 and AS1708727. The FOXO1 inhibitor may be present at a concentration of about 50 to 500 ng/mL, such as 100-200, 200-300, 300-400, or 400-500 ng/mL.

### 5. STAT6 Inhibitor

STAT6 inhibitors that may be used in the present methods include, but are not limited to, AS 1517499 and leflunomide (ALX-430-095). Small molecule peptide mimetics that target the SH2 domain of STAT6 are disclosed in U.S. Patent No. 6,426,331 and PCT Patent Publication No. WO2001/083517; both incorporated herein by reference. The STAT6 inhibitor may be present at a concentration of about 10 to 250 ng/mL, such as 20-50, 50-75, 75-100, 100-150, 150-200, or 200-250 ng/mL.

### 6. CpG Oligodeoxynucleotides

In certain embodiments, the B cells are expanded with CpG nucleotides. CpG oligodeoxynucleotides (ODN) are short single-stranded synthetic DNA molecules that contain a cytosine triphosphate deoxynucleotide followed by a guanine triphosphate deoxynucleotide which can act as immunostimulants. The CpG motifs are considered pathogen-associated molecular patterns (PAMPs) which are recognized by the pattern recognition receptor (PRR) Toll-like receptor 9 (TLR9) expressed on B cells and dendritic cells.

For facilitating uptake into cells, CpG containing oligonucleotides are preferably in the range of 8 to 100 bases in length. However, nucleic acids of any size greater than 8 nucleotides (even many kb long) are capable of inducing an immune response according to the invention if sufficient immunostimulatory motifs are present, since larger nucleic acids are degraded into oligonucleotides inside of cells. Preferably, the CpG oligonucleotide is in the range of between 8 and 100 and in some embodiments between 8 and 30 nucleotides in size. The CpG nucleic acid sequences may be as disclosed in International Publication Nos. WO2000/06588 and WO2000/06588 as well as U.S. Patent No. 7,488,490; all incorporated herein by reference. The entire CpG oligonucleotide can be unmethylated or portions may be unmethylated but at least the C of the 5' CG 3' must be unmethylated. One exemplary CpG oligonucleotide represented by at least the formula: 5'N₁X₁CGX₂N₂3' wherein at least one nucleotide separates consecutive CpGs; X₁ is adenine, guanine, or thymine; X₂ is cytosine, adenine, or thymine; N is any nucleotide and N₁ and N₂ are nucleic acid sequences composed of from about 0-25 N's each. An exemplary CpG ODN has the sequence 5' TCCAT-GACGTTCCTGATGCT 3' (SEQ ID NO:1). An additional exemplary CpG ODN is a 24-mer ODN 2006 that is able to modulate the immune response in both human and mice and has the sequence: 5'-tcgtcgttttgtcgttttgtcgtt-3' (SEQ ID NO:2) where regular letters represent PS linkage and bold letters represent CpG dinucleotides.

The expansion culture may comprise one or more distinct CpG ODN sequences at a concentration of 0.1 to 10 µg/mL, such as around 0.5, 1, 1.5, 2, 2.5, 3, 3.5, 4, 4.5, 5, 4.5, 6, 7, 8, 9, or 10 µg/mL of CpG ODNs, such as 0.1-2, 1-3, 2-4, 3-6, 4-7, 5-8, 7-9, or 8-10 µg/mL of Cpg ODNs. In particular aspects, the B cells are treated with about 3 µg/mL of CpG ODNs.

### III. Regulatory T Cells

### A. Starting Population of T Cells

The starting population of T cells may be isolated from subjects, particularly human subjects. The starting population of T cells can be isolated and expanded from a donor sample, such as an allogeneic sample, or from the subject who will receive the cells (*i.e.,* autologous). The starting population of T cells can be obtained from a subject of interest, such as a subject suspected of having a particular disease or condition, a subject suspected of having a predisposition to a particular disease or condition, or a subject who is undergoing therapy for a particular disease or condition. The starting population of T cells can be collected from any location in which they reside in the subject including, but not limited to, blood, cord blood, spleen, thymus, lymph nodes, and bone marrow. The isolated starting population of T cells may be used directly, or they can be stored for a period of time, such as by freezing.

The starting population of T cells may be enriched/purified from any tissue where they reside including, but not limited to, blood (including blood collected by blood banks or cord blood banks), spleen, bone marrow, tissues removed and/or exposed during surgical procedures, and tissues obtained via biopsy procedures. Tissues/organs from which the immune cells are enriched, isolated, and/or purified may be isolated from both living and non-living subjects, wherein the non-living subjects are organ donors.

In particular embodiments, the starting population of T cells are isolated from blood, such as peripheral blood or cord blood. In some aspects, starting population of T cells isolated from cord blood have enhanced immunomodulation capacity, such as measured by CD4- or CD8-positive T cell suppression. In specific aspects, the starting population of T cells are isolated from pooled blood, particularly pooled cord blood, for enhanced immunomodulation capacity. The pooled blood may be from 2 or more sources, such as 3, 4, 5, 6, 7, 8, 9, 10 or more sources (*e.g*., donor subjects).

When the population of immune cells is obtained from a donor distinct from the subject, the donor is preferably allogeneic, provided the cells obtained are subject-compatible in that they can be introduced into the subject. Allogeneic donor cells may or may not be human-leukocyte-antigen (HLA)-compatible. To be rendered subject-compatible, allogeneic cells can be treated to reduce immunogenicity.

In some aspects, the cells are human cells. The cells typically are primary cells, such as those isolated directly from a subject and/or isolated from a subject and frozen. In some embodiments, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, as described herein, and reintroducing them into the same patient, before or after cryopreservation.

Among the sub-types and subpopulations of T cells (*e.g.,* CD4⁺ and/or CD8⁺ T cells) are naive T (T_{N}) cells, effector T cells (T_{EFF}), memory T cells and sub-types thereof, such as stem cell memory T (TSC_{M}), central memory T (TC_{M}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some embodiments, one or more of the T cell populations is enriched for or depleted of cells that are positive for a specific marker, such as surface markers, or that are negative for a specific marker. In some cases, such markers are those that are absent or expressed at relatively low levels on certain populations of T cells (*e.g*., non-memory cells) but are present or expressed at relatively higher levels on certain other populations of T cells (*e.g.,* memory cells).

In particular embodiments, the isolated T cell population is a CD9⁺ T cell population. The present studies have shown that CD9 may be used as a marker for isolating and expanding T cells which are highly suppressive. In some aspects, the CD9⁺ T cell population is CD4⁺ and/or CD25⁺, particularly a CD4⁺CD25⁺CD9⁺ T cell population.

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

Generally, the starting cell populations are isolated from blood drawn from a subject, for example using apheresis (*e*.*g*., leukapheresis) or venous puncture. In one example, blood is obtained from a donor subject, such as an HLA-matched donor or the same subject who is to receive the antigen-specific T cells (recipient subject). In one example, an HLA-matched donor is one that matches at least 1/6, such as 2/6, 3/6, 4/6 or particularly 5/6 or 6/6, of the HLA loci (such as the A, B, and DR loci). In particular examples, the HLA-matched donor is a first degree relative. Monocytes can be isolated from blood obtained from the subject using methods known in the art. In one example, monocytes are obtained by elutriation of monocytes. In another example, monocytes are obtained from peripheral blood mononuclear cells (PBMCs) using a kit to deplete non-monocytic cells (for example from Miltenyi Biotec, Auburn, CA) or by positive selection using anti-CD 14 magnetic beads as recommended by the manufacturer (Miltenyi Biotec). In another example, PBMCs are prepared by centrifugation over a Ficoll-Paque (Pharmacia, Uppsala, Sweden) density gradient and the monocytes separated from lymphocytes by counterflow centrifugation (for example using the J6-MC elutriator system; Beckman Instruments, Palo Alto, CA) or centrifugation on a continuous Percoll (Pharmacia, Piscataway, NJ) density gradient.

Similarly, lymphocytes can be isolated from blood obtained from the subject using methods known in the art. In one example, lymphocytes are collected by elutriation of the lymphocytes. B cells can also be depleted. In another example, PBMCs are prepared by centrifugation over a Ficoll-Paque density gradient and the lymphocytes separated from monocytes as described above.

In some examples, a monocyte/lymphocyte population (a leukocyte pack or peripheral blood leukocytes (PBL)) is isolated from a subject. PBLs can be obtained by incubation of citrated blood in a medium that lyses erythrocytes, and removal of the lysed cells, thereby generating a PBL population. In one example, blood is incubated in NH₄Cl buffer (0.15 M NH₄Cl, 10 mM NaHCO₃ [pH 7.4]) for 5 minutes at 4°C (this can be repeated three times), followed by a wash in Ca²⁺-Mg²⁺-free phosphate-buffered saline (PBS-A) supplemented with 0.035% (wt/vol) EDTA and centrifugation to remove the lysed erythrocytes. However, this method is exemplary, and other methods known to those of skill in the art can also be utilized. The resultant monocyte, lymphocyte, or monocyte/lymphocyte product can be cryopreserved prior to use, using standard methods (for example using a combination of Pentastarch and DMSO). In some examples, cells are cryopreserved in aliquots of 5 to 200 × 10⁶ cells/vial, such as 6-10 × 10⁶ monocytes/vial, such as 50-200 × 10⁶ lymphocytes/vial, such as 10-50 × 10⁶ PBL/vial. To qualify for cryopreservation, the cell culture ideally contains predominately monocyte, lymphocyte, or monocyte/lymphocyte cells by flow cytometry. Sterility of the population need not be determined at this stage of the target antigen-specific T cells generation procedure; such a determination can occur after the final co-culture of cells. Methods for obtaining other APC populations, such as dendritic and B lymphoblastoid cells, are known in the art. For example, the Blood Dendritic Cell Isolation Kit II (Miltenyi Biotec Inc., Auburn, CA) can be used to obtain dendritic cells from blood according to the manufacturer's instructions or by culture from blood cells using the method of Wong *et al.,* 2002, herein incorporated by reference. B lymphoblastoid cells can be cultured from peripheral blood, for example using the method of Tosato (Coligan *et al,* 1994, herein incorporated by reference).

### B. Expansion of Regulatory T Cells

The expansion method for Tregs may comprise contacting the cells with a T cell receptor (TCR) activator (*e*.*g*., anti-CD3 antibody), a TCR co-stimulator (*e.g.,* anti-CD28 antibody), IL-2, and an mTOR inhibitor (*e.g*., rapamycin). The combination of the TCR activator and TCR co-stimulator are referred to as TCR ligation. The expansion culture may be for about 8-15 days, such as about 10-14 days, such as 10, 11, 12, 13, or 14 days.

The TCR co-stimulator may be an antibody or ligand for CD28, CD 137 (4-1BB), GITR, B7-1/2, CD5, ICOS, OX40 or CD40. The TCR activator and TCR co-stimulator may be immobilized to a solid phase. The antibodies may be provided at variable concentration on the solid support (*e.g.,* beads), such as at a ratio of about 1:10 to about 10:1 of CD28 antibody to TCR/CD3 antibody. More than one of the TCR/CD3 activators and/or more than one of the TCR co-stimulators may be used in the present methods.

Rapamycin is contacted with the cells prior to, simultaneously with, and/or subsequent to contact of the cells with the activators. Rapamycin is preferably present throughout the Treg expansion. The rapamycin may be added in one or more steps. Thus, for example, as described in the examples herein, isolated T cells may be stimulated with activators (CD3 antibodies and CD28 antibodies) and rapamycin at the same time. In this method, subsequent growth and passaging may be performed in the presence of rapamycin, but not the activators.

Rapamycin may be used at a concentration of from about 0.01 µM to about 10 µM, such as about 0.5 µM to about 2 µM, or about 1 µM. Rapamycin is a protein kinase inhibitor with a molecular weight of 914.2, also referred to as Sirolimus, Rapamune, AY-22989, RAPA and NSC-226080, available from Sigma, Calbio Chem, LC Labs etc. Rapamycin is available from a variety of commercial sources, such as A.G. Scientific, Inc. (Sa.n Diego, Calif., USA). In particular aspects, the rapamycin is present in the culture at a concentration of about 10 to 500 ng/mL, such as about 50 to 150 ng/mL, such as about 50, 75, 100, 125, or 150 ng/mL.

The Treg expansion culture may comprise IL-2 at a concentration of about 50 to 1000 IU/mL, such as about 100 to 250 IU/mL, 200 to 400 IU/mL, 300 to 400 IU/mL, 350 to 450 IU/mL, 400 to 600 IU/mL, 450 to 550 IU/mL, 500 to 750 IU/mL, or 700 to 1000 IU/mL.

The expansion culture may comprise adenosine receptor (A2AR) or its agonist, TNFR2 or its agonist, and/or all-trans retinoic acid (ATRA) for further enhancement of Treg expansion. The A2AR, TNFR2, or their agonists may be present in the culture at a concentration of about 0.5 to 5 µg/mL, such as about 1.0 to 3.0 µg/mL, 2.0 to 2.5 µg/mL, 2.2 to 2.8 µg/mL, 3.0 to 3.5 µg/mL, 3.5 to 4.0 µg/mL, or 4.0 to 5.0 µg/mL. The ATRA may be present in the culture at a concentration of about 1 to 50 nM, such as about 5 to 15 nM, 10 to 20 nM, 15 to 25 nM, 20 to 30 nM, 30 to 40 nM, or 40 to 50 nM.

Expanded Tregs, such as those expanded by the methods provided in the present disclosure or by other methods known in the art, may be subjected to a selection step for the isolation of CD9⁺ Tregs with suppressive activity. The selection of CD9⁺ Tregs may be performed by methods known in the art. For example, positive or negative selection may be employed for the selection of CD9⁺ Tregs. Selection may comprise centrifugation, cell elutriation, magnetic separation, adhesion, complement lysis or flow cytometry. In one method, cell sorting, such as FACS or MACS, is used to isolate CD9⁺ T cells or remove CD9⁻ T cells.

The Tregs produced herein are able to suppress proliferation of syngeneic T cells *in vitro.* At a 1:1 ratio of the test T cells, with, for example, T cells prior to expansion, immunosuppressive cells preferably achieve at least 50, 60, 70, 80 or 90% suppression of proliferation, *i*.*e*. reduction in cell numbers relative to control.

### C. Genetically Engineered T Cells

The Tregs may be genetically engineered to express antigen receptors such as engineered TCRs and/or chimeric antigen receptors (CARs). For example, the host cells (*e.g*. autologous or allogeneic T-cells) are modified to express a T cell receptor (TCR) having antigenic specificity for a cancer antigen. Multiple CARs and/or TCRs, such as to different antigens, may be added to the T cells.

Suitable methods of modification are known in the art. See, for instance, Sambrook and Ausubel, supra. For example, the cells may be transduced to express a T cell receptor (TCR) having antigenic specificity for a cancer antigen using transduction techniques described in Heemskerk *et al.,* 2008 and Johnson *et al.,* 2009.

Electroporation of RNA coding for the full length TCR α and β (or γ and δ) chains can be used as alternative to overcome long-term problems with autoreactivity caused by pairing of retrovirally transduced and endogenous TCR chains. Even if such alternative pairing takes place in the transient transfection strategy, the possibly generated autoreactive T cells will lose this autoreactivity after some time, because the introduced TCR α and β chain are only transiently expressed. When the introduced TCR α and β chain expression is diminished, only normal autologous T cells are left. This is not the case when full length TCR chains are introduced by stable retroviral transduction, which will never lose the introduced TCR chains, causing a constantly present autoreactivity in the patient.

In some embodiments, the cells comprise one or more nucleic acids introduced via genetic engineering that encode one or more antigen receptors, and genetically engineered products of such nucleic acids. In some embodiments, the nucleic acids are heterologous, *i*.*e*., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature (*e.g.,* chimeric).

In some embodiments, the CAR contains an extracellular antigen-recognition domain that specifically binds to an antigen. In some embodiments, the antigen is a protein expressed on the surface of cells. In some embodiments, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs and recombinant TCRs, as well as methods for engineering and introducing the receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain et al., Cancer Discov. 2013; 3(4): 388-398; Davila et al. (2013) PLoS ONE 8(4): e61338; Turtle et al., Curr. Opin. Immunol., 2012; 24(5): 633-39; Wu et al., Cancer, 2012, 18(2): 160-75. In some aspects, the genetically engineered antigen receptors include a CAR as described in U.S. Patent No.: 7,446,190, and those described in International Patent Application Publication No.: WO/2014055668 A1.

### 2. Chimeric Antigen Receptors

In some embodiments, the chimeric antigen receptor comprises: a) an intracellular signaling domain, b) a transmembrane domain, and c) an extracellular domain comprising an antigen binding region.

In some embodiments, the engineered antigen receptors include CARs, including activating or stimulatory CARs, costimulatory CARs (see WO2014/055668), and/or inhibitory CARs (iCARs, see Fedorov *et al.,* 2013). The CARs generally include an extracellular antigen (or ligand) binding domain linked to one or more intracellular signaling components, in some aspects via linkers and/or transmembrane domain(s). Such molecules typically mimic or approximate a signal through a natural antigen receptor, a signal through such a receptor in combination with a costimulatory receptor, and/or a signal through a costimulatory receptor alone.

Certain embodiments of the present disclosure concern the use of nucleic acids, including nucleic acids encoding an antigen-specific chimeric antigen receptor (CAR) polypeptide, including a CAR that has been humanized to reduce immunogenicity (hCAR), comprising an intracellular signaling domain, a transmembrane domain, and an extracellular domain comprising one or more signaling motifs. In certain embodiments, the CAR may recognize an epitope comprising the shared space between one or more antigens. In certain embodiments, the binding region can comprise complementary determining regions of a monoclonal antibody, variable regions of a monoclonal antibody, and/or antigen binding fragments thereof. In another embodiment, that specificity is derived from a peptide (*e*.*g*., cytokine) that binds to a receptor.

It is contemplated that the human CAR nucleic acids may be human genes used to enhance cellular immunotherapy for human patients. In a specific embodiment, the invention includes a full-length CAR cDNA or coding region. The antigen binding regions or domain can comprise a fragment of the V_{H} and V_{L} chains of a single-chain variable fragment (scFv) derived from a particular human monoclonal antibody, such as those described in U.S. Patent 7,109,304, incorporated herein by reference. The fragment can also be any number of different antigen binding domains of a human antigen-specific antibody. In a more specific embodiment, the fragment is an antigen-specific scFv encoded by a sequence that is optimized for human codon usage for expression in human cells.

The arrangement could be multimeric, such as a diabody or multimers. The multimers are most likely formed by cross pairing of the variable portion of the light and heavy chains into a diabody. The hinge portion of the construct can have multiple alternatives from being totally deleted, to having the first cysteine maintained, to a proline rather than a serine substitution, to being truncated up to the first cysteine. The Fc portion can be deleted. Any protein that is stable and/or dimerizes can serve this purpose. One could use just one of the Fc domains, *e.g.,* either the CH2 or CH3 domain from human immunoglobulin. One could also use the hinge, CH2 and CH3 region of a human immunoglobulin that has been modified to improve dimerization. One could also use just the hinge portion of an immunoglobulin. One could also use portions of CD8alpha.

In some embodiments, the CAR nucleic acid comprises a sequence encoding other costimulatory receptors, such as a transmembrane domain and a modified CD28 intracellular signaling domain. Other costimulatory receptors include, but are not limited to one or more of CD28, CD27, OX-40 (CD134), DAP10, and 4-1BB (CD137). In addition to a primary signal initiated by CD3ζ, an additional signal provided by a human costimulatory receptor inserted in a human CAR is important for full activation of NK cells and could help improve *in vivo* persistence and the therapeutic success of the adoptive immunotherapy.

In some embodiments, CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (VH) and variable light (VL) chains of a monoclonal antibody (mAb).

In certain embodiments of the chimeric antigen receptor, the antigen-specific portion of the receptor (which may be referred to as an extracellular domain comprising an antigen binding region) comprises a tumor associated antigen or a pathogen-specific antigen binding domain. Antigens include carbohydrate antigens recognized by pattern-recognition receptors, such as Dectin-1. A tumor associated antigen may be of any kind so long as it is expressed on the cell surface of tumor cells. Exemplary embodiments of tumor associated antigens include CD19, CD20, carcinoembryonic antigen, alphafetoprotein, CA-125, MUC-1, CD56, EGFR, c-Met, AKT, Her2, Hers, epithelial tumor antigen, melanoma-associated antigen, mutated p53, mutated ras, and so forth. In certain embodiments, the CAR may be co-expressed with a cytokine to improve persistence when there is a low amount of tumor-associated antigen. For example, CAR may be co-expressed with IL-15.

The sequence of the open reading frame encoding the chimeric receptor can be obtained from a genomic DNA source, a cDNA source, or can be synthesized (*e.g., via* PCR), or combinations thereof. Depending upon the size of the genomic DNA and the number of introns, it may be desirable to use cDNA or a combination thereof as it is found that introns stabilize the mRNA. Also, it may be further advantageous to use endogenous or exogenous non-coding regions to stabilize the mRNA.

It is contemplated that the chimeric construct can be introduced into immune cells as naked DNA or in a suitable vector. Methods of stably transfecting cells by electroporation using naked DNA are known in the art. See, *e.g.,* U.S. Patent No. 6,410,319. Naked DNA generally refers to the DNA encoding a chimeric receptor contained in a plasmid expression vector in proper orientation for expression.

Alternatively, a viral vector (*e*.*g*., a retroviral vector, adenoviral vector, adeno-associated viral vector, or lentiviral vector) can be used to introduce the chimeric construct into immune cells. Suitable vectors for use in accordance with the method of the present disclosure are non-replicating in the immune cells. A large number of vectors are known that are based on viruses, where the copy number of the virus maintained in the cell is low enough to maintain the viability of the cell, such as, for example, vectors based on HIV, SV40, EBV, HSV, or BPV.

In some aspects, the antigen-specific binding, or recognition component is linked to one or more transmembrane and intracellular signaling domains. In some embodiments, the CAR includes a transmembrane domain fused to the extracellular domain of the CAR. In one embodiment, the transmembrane domain that naturally is associated with one of the domains in the CAR is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (*i.e.* comprise at least the transmembrane region(s) of) the alpha, beta or zeta chain of the T- cell receptor, CD28, CD3 zeta, CD3 epsilon, CD3 gamma, CD3 delta, CD45, CD4, CD5, CD8, CD9, CD 16, CD22, CD33, CD37, CD64, CD80, CD86, CD 134, CD137, CD154, ICOS/CD278, GITR/CD357, NKG2D, and DAP molecules. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain.

In certain embodiments, the platform technologies disclosed herein to genetically modify immune cells comprise (i) non-viral gene transfer using an electroporation device (*e.g.,* a nucleofector), (ii) CARs that signal through endodoinains (*e.g.,* CD28/CD3-ζ, C137/CD3-ζ, or other combinations), (iii) CARs with variable lengths of extracellular domains connecting the antigen-recognition domain to the cell surface, and, in some cases, (iv) artificial antigen presenting cells (aAPC) derived from K562 to be able to robustly and numerically expand CAR" immune cells.

### 3. T Cell Receptor (TCR)

In some embodiments, the genetically engineered antigen receptors include recombinant T cell receptors (TCRs) and/or TCRs cloned from naturally occurring T cells. A "T cell receptor" or "TCR" refers to a molecule that contains a variable a and β chains (also known as TCRα and TCRβ, respectively) or a variable γ and δ chains (also known as TCRγ and TCRδ, respectively) and that is capable of specifically binding to an antigen peptide bound to a MHC receptor. In some embodiments, the TCR is in the αβ form.

Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules. In some embodiments, a TCR also can contain a constant domain, a transmembrane domain and/or a short cytoplasmic tail (see, *e.g.,* Janeway *et al.* 1997). For example, in some aspects, each chain of the TCR can possess one N-terminal immunoglobulin variable domain, one immunoglobulin constant domain, a transmembrane region, and a short cytoplasmic tail at the C- terminal end. In some embodiments, a TCR is associated with invariant proteins of the CD3 complex involved in mediating signal transduction.

Thus, for purposes herein, reference to a TCR includes any TCR or functional fragment, such as an antigen-binding portion of a TCR that binds to a specific antigenic peptide bound in an MHC molecule, *i.e.* MHC-peptide complex. An "antigen-binding portion" or antigen- binding fragment" of a TCR, which can be used interchangeably, refers to a molecule that contains a portion of the structural domains of a TCR, but that binds the antigen (*e.g*. MHC-peptide complex) to which the full TCR binds. In some cases, an antigen-binding portion contains the variable domains of a TCR, such as variable a chain and variable β chain of a TCR, sufficient to form a binding site for binding to a specific MHC-peptide complex, such as generally where each chain contains three complementarity determining regions.

In some embodiments, the variable domains of the TCR chains associate to form loops, or complementarity determining regions (CDRs) analogous to immunoglobulins, which confer antigen recognition and determine peptide specificity by forming the binding site of the TCR molecule and determine peptide specificity. Typically, like immunoglobulins, the CDRs are separated by framework regions (FRs) (Lefranc *et al.,* 2003). In some embodiments, CDR3 is the main CDR responsible for recognizing processed antigen, although CDR1 of the alpha chain has also been shown to interact with the N-terminal part of the antigenic peptide, whereas CDR1 of the beta chain interacts with the C-terminal part of the peptide. CDR2 is thought to recognize the MHC molecule. In some embodiments, the variable region of the β-chain can contain a further hypervariability (HV4) region.

In some embodiments, the TCR chains contain a constant domain. For example, like immunoglobulins, the extracellular portion of TCR chains (*e.g*., a-chain, β-chain) can contain two immunoglobulin domains, a variable domain (*e.g.*, Vₐ or Vp; typically amino acids 1 to 116 based on Kabat numbering Kabat et al., "Sequences of Proteins of Immunological Interest, US Dept. Health and Human Services, Public Health Service National Institutes of Health, 1991, 5th ed.) at the N-terminus, and one constant domain (*e.g.,* a-chain constant domain or Cₐ, typically amino acids 117 to 259 based on Kabat, β-chain constant domain or Cp, typically amino acids 117 to 295 based on Kabat) adjacent to the cell membrane. For example, in some cases, the extracellular portion of the TCR formed by the two chains contains two membrane-proximal constant domains, and two membrane-distal variable domains containing CDRs. The constant domain of the TCR domain contains short connecting sequences in which a cysteine residue forms a disulfide bond, making a link between the two chains. In some embodiments, a TCR may have an additional cysteine residue in each of the a, and β chains such that the TCR contains two disulfide bonds in the constant domains.

In some embodiments, the TCR chains can contain a transmembrane domain. In some embodiments, the transmembrane domain is positively charged. In some cases, the TCR chains contains a cytoplasmic tail. In some cases, the structure allows the TCR to associate with other molecules like CD3. For example, a TCR containing constant domains with a transmembrane region can anchor the protein in the cell membrane and associate with invariant subunits of the CD3 signaling apparatus or complex.

Generally, CD3 is a multi-protein complex that can possess three distinct chains (y, δ, and ε) in mammals and the ζ-chain. For example, in mammals the complex can contain a CD3γ chain, a CD3δ chain, two CD3ε chains, and a homodimer of CD3ζ chains. The CD3γ, CD3δ, and CD3ε chains are highly related cell surface proteins of the immunoglobulin superfamily containing a single immunoglobulin domain. The transmembrane regions of the CD3γ, CD3δ, and CD3ε chains are negatively charged, which is a characteristic that allows these chains to associate with the positively charged T cell receptor chains. The intracellular tails of the CD3γ, CD3δ, and CD3ε chains each contain a single conserved motif known as an immunoreceptor tyrosine -based activation motif or ITAM, whereas each CD3ζ chain has three. Generally, ITAMs are involved in the signaling capacity of the TCR complex. These accessory molecules have negatively charged transmembrane regions and play a role in propagating the signal from the TCR into the cell. The CD3- and ζ-chains, together with the TCR, form what is known as the T cell receptor complex.

In some embodiments, the TCR may be a heterodimer of two chains α and β (or optionally γ and δ) or it may be a single chain TCR construct. In some embodiments, the TCR is a heterodimer containing two separate chains (α, and β chains or γ and δ chains) that are linked, such as by a disulfide bond or disulfide bonds. In some embodiments, a TCR for a target antigen (*e.g.*, a cancer antigen) is identified and introduced into the cells. In some embodiments, nucleic acid encoding the TCR can be obtained from a variety of sources, such as by polymerase chain reaction (PCR) amplification of publicly available TCR DNA sequences. In some embodiments, the TCR is obtained from a biological source, such as from cells such as from a T cell (*e.g.* cytotoxic T cell), T-cell hybridomas or other publicly available source. In some embodiments, the T-cells can be obtained from *in vivo* isolated cells. In some embodiments, a high-affinity T cell clone can be isolated from a patient, and the TCR isolated. In some embodiments, the T- cells can be a cultured T-cell hybridoma or clone. In some embodiments, the TCR clone for a target antigen has been generated in transgenic mice engineered with human immune system genes (*e.g*., the human leukocyte antigen system, or HLA). See, *e.g*., tumor antigens (see, *e.g.*, Parkhurst *et al.,* 2009; Cohen *et al.,* 2005). In some embodiments, phage display is used to isolate TCRs against a target antigen (see, *e.g*., Varela-Rohena *et al.,* 2008, Li, 2005;). In some embodiments, the TCR or antigen-binding portion thereof can be synthetically generated from knowledge of the sequence of the TCR.

### 4. Suicide Genes

The CAR and/or TCR of the T cells of the present disclosure may comprise one or more suicide genes. The term "suicide gene" as used herein is defined as a gene which may be used to selectively target cells for killing. For example, as suicide gene may, upon administration of a prodrug, effect transition of a gene product to a compound which kills its host cell. Examples of suicide gene/prodrug combinations which may be used are Herpes Simplex Virus-thymidine kinase (HSV-tk) and ganciclovir, acyclovir, or F1AU; oxidoreductase and cycloheximide; cytosine deaminase and 5-fluorocytosine; thymidine kinase thymidilate kinase (Tdk::Tmk) and AZT; and deoxycytidine kinase and cytosine arabinoside.

The *E.coli* purine nucleoside phosphorylase, a so-called suicide gene which converts the prodrug 6-methylpurine deoxyriboside to toxic purine 6-methylpurine. Other examples of suicide genes used with prodrug therapy are the *E*. *coli* cytosine deaminase gene and the HSV thymidine kinase gene.

Exemplary suicide genes include CD20, CD52, EGFRv3, or inducible caspase 9. In one embodiment, a truncated version of EGFR variant III (EGFRv3) may be used as a suicide antigen which can be ablated by Cetuximab. Further suicide genes known in the art that may be used in the present disclosure include Purine nucleoside phosphorylase (PNP), Cytochrome p450 enzymes (CYP), Carboxypeptidases (CP), Carboxylesterase (CE), Nitroreductase (NTR), Guanine Ribosyltransferase (XGRTP), Glycosidase enzymes, Methionine-α,γ-lyase (MET), and Thymidine phosphorylase (TP).

### 5. Modification of Gene Expression

In some embodiments, the cells of the present disclosure are modified to have altered expression of certain genes such as glucocorticoid receptor, TGFβ receptor (e.g., TGFβ-RII), and/or CISH. In one embodiment, the cells may be modified to express a dominant negative TGFβ receptor II (TGFβRIIDN) which can function as a cytokine sink to deplete endogenous TGFβ.

In some embodiments, the altered gene expression is carried out by effecting a disruption in the gene, such as a knock-out, insertion, missense or frameshift mutation, such as biallelic frameshift mutation, deletion of all or part of the gene, *e.g.,* one or more exon or portion therefore, and/or knock-in. For example, the altered gene expression can be effected by sequence-specific or targeted nucleases, including DNA-binding targeted nucleases such as zinc finger nucleases (ZFN) and transcription activator-like effector nucleases (TALENs), and RNA-guided nucleases such as a CRISPR-associated nuclease (Cas), specifically designed to be targeted to the sequence of the gene or a portion thereof.

Exemplary gRNA sequences for CRISPR-Cas mediated knockdown of NR3CS (glucocorticoid receptor) include Ex3 NR3C1 sG1 5-TGC TGT TGA GGA GCT GGA-3 (SEQ ID NO:3) and Ex3 NR3C1 sG2 5-AGC ACA CCA GGC AGA GTT-3 (SEQ ID NO:4). Exemplary gRNA sequences for TGF-beta receptor 2 include EX3 TGFBR2 sG1 5-CGG CTG AGG AGC GGA AGA-3 (SEQ ID NO:5) and EX3 TGFBR2 sG2 5-TGG-AGG-TGA-GCA-ATC-CCC-3 (SEQ ID NO:6). The T7 promoter, target sequence, and overlap sequence may have the sequence TTAATACGACTCACTATAGG (SEQ ID NO:7) + target sequence + gttttagagctagaaatagc (SEQ ID NO:8).

### IV. Methods of Use

Certain embodiments of the present disclosure concern methods for the use of the Breg and/or Treg populations provided herein for treating or preventing an inflammatory or immune-mediated disorder. The method includes administering to the subject a therapeutically effective amount of the Bregs and/or Tregs, thereby treating or preventing the inflammatory or immune-mediated disorder in the subject.

The Tregs and/or Bregs generated according to the present methods have many potential uses, including experimental and therapeutic uses. In particular, it is envisaged that such cell populations will be extremely useful in suppressing undesirable or inappropriate immune responses. In such methods, a small number of T cells and/or Bcells are removed from a patient and then manipulated and expanded *ex vivo* before reinfusing them into the patient. Examples of diseases which may be treated in this way are autoimmune diseases and conditions in which suppressed immune activity is desirable, *e.g*., for allo-transplantation tolerance. A therapeutic method could comprise providing a mammal, obtaining T cells and/or B cells from the mammal; expanding the T cells and/or B cells *ex vivo* in accordance with the methods of the present methods as described above; and administering the expanded T regs and/or Bregs to the mammal to be treated.

In one embodiment, a subject suffering from an autoimmune disease or an inflammatory disease (*e.g.*, associated with diminished levels of IL-10) is administered a population of T regs and/or Bregs. In one aspect of this embodiment, the B cell and/or T cell population is isolated from the patient themselves, *i.e*., the subject is the donor. In another aspect of this embodiment, the B cell and/or T cell population is isolated from a donor that is not the subject. In an aspect of this embodiment, the B cell and/or T cell population is pooled from several donors, such as from the cord blood of several donors. According to this embodiment, administration of a regulatory B cell population to a subject in need thereof results in an increased level ofIL-10 production in the patient sufficient to control, reduce, or eliminate symptoms of the disease being treated.

In some embodiments, the Treg and/or Breg populationg is contacted with an antigen specific to a disorder, such as an autoimmune disorder, prior to introducing them to a subject. For example, the regulatory cells may be exposed to an autoantigen such as insulin or GAD-65 prior to administration to a subject to prevent or treat diabetes.

In one embodiment, the subject has an autoimmune disease. Non-limiting examples of autoimmune diseases include: alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac spate-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Meniere's disease, mixed connective tissue disease, multiple sclerosis, type 1 or immune-mediated diabetes mellitus, myasthenia gravis, nephrotic syndrome (such as minimal change disease, focal glomerulosclerosis, or mebranous nephropathy), pemphigus vulgaris, pernicious anemia, polyarteritis nodosa, polychondritis, polyglandular syndromes, polymyalgia rheumatics, polymyositis and dermatomyositis, primary agammaglobulinemia, primary biliary cirrhosis, psoriasis, psoriatic arthritis, Raynaud's phenomenon, Reiter's syndrome, Rheumatoid arthritis, sarcoidosis, scleroderma, Sjogren's syndrome, stiff-man syndrome, systemic lupus erythematosus, lupus erythematosus, ulcerative colitis, uveitis, vasculitides (such as polyarteritis nodosa, takayasu arteritis, temporal arteritis/giant cell arteritis, or dermatitis herpetiformis vasculitis), vitiligo, and Wegener's granulomatosis. Thus, some examples of an autoimmune disease that can be treated using the methods disclosed herein include, but are not limited to, multiple sclerosis, rheumatoid arthritis, systemic lupus erythematosis, type T diabetes mellitus, Crohn's disease; ulcerative colitis, myasthenia gravis, glomerulonephritis, ankylosing spondylitis, vasculitis, or psoriasis. The subject can also have an allergic disorder such as Asthma.

In yet another embodiment, the subject is the recipient of a transplanted organ or stem cells and expanded regulatory cells (*e*.*g*., Tregs and/or Bregs) are used to prevent and/or treat rejection. In particular embodiments, the subject has or is at risk of developing graft versus host disease. GVHD is a possible complication of any transplant that uses or contains stem cells from either a related or an unrelated donor. There are two kinds of GVHD, acute and chronic. Acute GVHD appears within the first three months following transplantation. Signs of acute GVHD include a reddish skin rash on the hands and feet that may spread and become more severe, with peeling or blistering skin. Acute GVHD can also affect the stomach and intestines, in which case cramping, nausea, and diarrhea are present. Yellowing of the skin and eyes (jaundice) indicates that acute GVHD has affected the liver. Chronic GVHD is ranked based on its severity: stage/grade 1 is mild; stage/grade 4 is severe. Chronic GVHD develops three months or later following transplantation. The symptoms of chronic GVHD are similar to those of acute GVHD, but in addition, chronic GVHD may also affect the mucous glands in the eyes, salivary glands in the mouth, and glands that lubricate the stomach lining and intestines. Any of the populations of regulatory B cells disclosed herein can be utilized. Examples of a transplanted organ include a solid organ transplant, such as kidney, liver, skin, pancreas, lung and/or heart, or a cellular transplant such as islets, hepatocytes, myoblasts, bone marrow, or hematopoietic or other stem cells. The transplant can be a composite transplant, such as tissues of the face. Regulatory B cells, such as immunosuppressive CD19⁺ cells, can be administered prior to transplantation, concurrently with transplantation, or following transplantation. In some embodiments, the regulatory B cells are administered prior to the transplant, such as at least 1 hour, at least 12 hours, at least 1 day, at least 2 days, at least 3 days, at least 4 days, at least 5 days, at least 6 days, at least 1 week, at least 2 weeks, at least 3 weeks, at least 4 weeks, or at least 1 month prior to the transplant. In one specific, non-limiting example, administration of the therapeutically effective amount of regulatory B cells occurs 3-5 days prior to transplantation.

A regulatory cell subset administered to a patient that is receiving a transplant can be sensitized with antigens specific to the transplanted material prior to administration. According to this embodiment, the transplant recipient will have a decreased immune/inflammatory response to the transplanted material and, as such, the likelihood of rejection of the transplanted tissue is minimized. Similarly, with regard to the treatment of graft versus host disease, the regulatory cell subset can be sensitized with antigens specific to the host. According to this embodiment, the recipient will have a decreased immune/inflammatory response to self-antigens.

In a further embodiment, administration of a therapeutically effective amount of regulatory cells (*e.g*., Tregs and/or Bregs) to a subject treats or inhibits inflammation in the subject. Thus, the method includes administering a therapeutically effective amount of regulatory cells to the subject to inhibit the inflammatory process. Examples of inflammatory disorders include, but are not limited to, asthma, encephalitis, inflammatory bowel disease, chronic obstructive pulmonary disease (COPD), allergic disorders, septic shock, pulmonary fibrosis, undifferentiated spondyloarthropathy, undifferentiated arthropathy, arthritis, inflammatory osteolysis, and chronic inflammation resulting from chronic viral or bacterial infections. The methods disclosed herein can also be used to treat allergic disorders.

Administration of regulatory cells can be utilized whenever immunosuppression or inhibition of inflammation is desired, for example, at the first sign or symptoms of a disease or inflammation. These may be general, such as pain, edema, elevated temperature, or may be specific signs or symptoms related to dysfunction of affected organ(s). For example in renal transplant rejection there may be an elevated serum creatinine level, whereas in GVHD, there may be a rash, and in asthma, there may be shortness of breath and wheezing.

Administration of regulatory cells can also be utilized to prevent immune-mediated disease in a subject of interest. For example, regulatory cells can be administered to a subject that will be a transplant recipient prior to the transplantation. In another example, regulatory cells are administered to a subject receiving allogeneic bone marrow transplants without T cell depletion. In a further example, regulatory cells can be administered to a subject with a family history of diabetes. In other example, regulatory cells are administered to a subject with asthma in order to prevent an asthma attack. In some embodiments, a therapeutically effective amount of regulatory cells is administered to the subject in advance of a symptom. The administration of the regulatory cells results in decreased incidence or severity of subsequent immunological event or symptom (such as an asthma attack), or improved patient survival, compared to patients who received other therapy not including regulatory cells.

The effectiveness of treatment can be measured by many methods known to those of skill in the art. In one embodiment, a white blood cell count (WBC) is used to determine the responsiveness of a subject's immune system. A WBC measures the number of white blood cells in a subject. Using methods well known in the art, the white blood cells in a subject's blood sample are separated from other blood cells and counted. Normal values of white blood cells are about 4,500 to about 10,000 white blood cells/µl. Lower numbers of white blood cells can be indicative of a state of immunosuppression in the subject.

In another embodiment, immunosuppression in a subject may be determined using a T-lymphocyte count. Using methods well known in the art, the white blood cells in a subject's blood sample are separated from other blood cells. T-lymphocytes are differentiated from other white blood cells using standard methods in the art, such as, for example, immunofluorescence or FACS. Reduced numbers of T-cells, or a specific population of T-cells can be used as a measurement of immunosuppression. A reduction in the number of T-cells, or in a specific population of T-cells, compared to the number of T-cells (or the number of cells in the specific population) prior to treatment can be used to indicate that immunosuppression has been induced.

In additional embodiments, tests to measure T cell activation, proliferation, or cytokine responses including those to specific antigens are performed. In some examples, the number of Treg or Breg cells can be measured in a sample from a subject. In additional examples, cytokines are measured in a sample, from a subject, such as IL-10.

In other examples, to assess inflammation, neutrophil infiltration at the site of inflammation can be measured. In order to assess neutrophil infiltration myeloperoxidase activity can be measured. Myeloperoxidase is a hemoprotein present in azurophilic granules of polymorphonuclear leukocytes and monocytes. It catalyzes the oxidation of halide ions to their respective hypohalous acids, which are used for microbial killing by phagocytic cells. Thus, a decrease in myeloperoxidase activity in a tissue reflects decreased neutrophil infiltration, and can serve as a measure of inhibition of inflammation.

In another example, effective treatment of a subject can be assayed by measuring cytokine levels in the subject. Cytokine levels in body fluids or cell samples are determined by conventional methods. For example, an immunospot assay, such as the enzyme-linked immunospot or "ELISPOT" assay, can be used. The immunospot assay is a highly sensitive and quantitative assay for detecting cytokine secretion at the single cell level. Immunospot methods and applications are well known in the art and are described, for example, in EP 957359. Variations of the standard immunospot assay are well known in the art and can be used to detect alterations in cytokine production in the methods of the disclosure (see, for example, U.S. Patent No. 5,939,281 and U.S. Patent No. 6,218,132).

In another embodiment, administration of a therapeutically effective amount of stimulated regulatory B cells to a subject induces the production or activity of regulatory T cells, such as CD4⁺CD25⁺ of CD4+Foxp3+ suppressive T cells. In further embodiments, administration of a therapeutically effective amount of stimulated regulatory B cells decreases the proliferation of CD4⁺ and/or CD8⁺ T cells. In further embodiments, administration of a therapeutically effective amount of stimulated regulatory B cells reduces production of antibodies produced by the subject's non-regulatory B cells that are involved in the immune-mediated disease. In further embodiments, regulatory B cells may inhibit influx of inflammatory cells or damage mediated by innate immune cells. Thus, all of these cell types can be measured. In a further embodiment, cytokine production can be measured.

Suppression of proliferation can be evaluated using many methods well known in the art. In one embodiment, cell proliferation is quantified by measuring [³H]-thymidine incorporation. Proliferating cells incorporate the labeled DNA precursor into newly synthesized DNA, such that the amount of incorporation, measured by liquid scintillation counting, is a relative measure of cellular proliferation. In another embodiment, cell proliferation is quantified using the thymidine analogue 5-bromo-2'-deoxyuridine (BrdU) in a proliferation assay. BrdU is incorporated into cellular DNA in a manner similar to thymidine, and is quantified using anti-BrdU mAbs by flow cytometry.

Therapeutically effective amounts of regulatory B cells can be administered by a number of routes, including parenteral administration, for example, intravenous, intraperitoneal, intramuscular, intrastemal, or intraarticular injection, or infusion.

The therapeutically effective amount of regulatory cells for use in inducing immunosuppression or treating or inhibiting inflammation is that amount that achieves a desired effect in a subject being treated. For instance, this can be the amount of regulatory cells necessary to inhibit advancement, or to cause regression of an autoimmune or alloimmune disease, or which is capable of relieving symptoms caused by an autoimmune disease, such as pain and inflammation. It can be the amount necessary to relieve symptoms associated with inflammation, such as pain, edema and elevated temperature. It can also be the amount necessary to diminish or prevent rejection of a transplanted organ.

The regulatory cell population can be administered in treatment regimens consistent with the disease, for example a single or a few doses over one to several days to ameliorate a disease state or periodic doses over an extended time to inhibit disease progression and prevent disease recurrence. The precise dose to be employed in the formulation will also depend on the route of administration, and the seriousness of the disease or disorder, and should be decided according to the judgment of the practitioner and each patient's circumstances. The therapeutically effective amount of regulatory cells will be dependent on the subject being treated, the severity and type of the affliction, and the manner of administration. In some embodiments, doses that could be used in the treatment of human subjects range from at least 3.8×10⁴, at least 3.8×10⁵, at least 3.8×10⁶, at least 3.8×10⁷, at least 3.8×10⁸, at least 3.8×10⁹, or at least 3.8×10¹⁰ regulatory cells/m². In a certain embodiment, the dose used in the treatment of human subjects ranges from about 3.8×10⁹ to about 3.8×10¹⁰ regulatory cells/m². In additional embodiments, a therapeutically effective amount of regulatory cells can vary from about 5×10⁶ cells per kg body weight to about 7.5×10⁸ cells per kg body weight, such as about 2×10⁷ cells to about 5×10⁸ cells per kg body weight, or about 5×10⁷ cells to about 2×10¹ cells per kg body weight. The exact amount of regulatory cells is readily determined by one of skill in the art based on the age, weight, sex, and physiological condition of the subject. Effective doses can be extrapolated from dose-response curves derived from in vitro or animal model test systems.

The expanded regulatory T and/or B cells may be administered in combination with one or more other therapeutic agents for the treatment of the immune-mediated disorder. Combination therapies can include, but are not limited to, one or more antimicrobial agents (for example, antibiotics, anti-viral agents and anti-fungal agents), anti-tumor agents (for example, fluorouracil, methotrexate, paclitaxel, fludarabine, etoposide, doxorubicin, or vincristine), immune-depleting agents (for example, fludarabine, etoposide, doxorubicin, or vincristine), immunosuppressive agents (for example, azathioprine, or glucocorticoids, such as dexamethasone or prednisone), anti-inflammatory agents (for example, glucocorticoids such as hydrocortisone, dexamethasone or prednisone, or non-steroidal anti-inflammatory agents such as acetylsalicylic acid, ibuprofen or naproxen sodium), cytokines (for example, interleukin-10 or transforming growth factor-beta), hormones (for example, estrogen), or a vaccine. In addition, immunosuppressive or tolerogenic agents including but not limited to calcineurin inhibitors (*e.g.* cyclosporin and tacrolimus); mTOR inhibitors (*e.g.* Rapamycin); mycophenolate mofetil, antibodies (*e.g.* recognizing CD3, CD4, CD40, CD154, CD45, IVIG, or B cells); chemotherapeutic agents (*e.g.* Methotrexate, Treosulfan, Busulfan); irradiation; or chemokines, interleukins or their inhibitors (*e.g.* BAFF, IL-2, anti-IL-2R, IL-4, JAK kinase inhibitors) can be administered. Such additional pharmaceutical agents can be administered before, during, or after administration of the regulatory B cells, depending on the desired effect. This administration of the cells and the agent can be by the same route or by different routes, and either at the same site or at a different site.

### V. Kits

In some embodiments, a kit that can include, for example, one or more media and components for the production of regulatory immune cells is provided. Such formulations may comprise a cocktail of factors, in a form suitable for combining with B cells or T cells. The reagent system may be packaged either in aqueous media or in lyophilized form, where appropriate. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a vial. The components of the kit may be provided as dried powder(s). When reagents and/or components are provided as a dry powder, the powder can be reconstituted by the addition of a suitable solvent. It is envisioned that the solvent may also be provided in another container means. The kits also will typically include a means for containing the kit component(s) in close confinement for commercial sale. Such containers may include injection or blow molded plastic containers into which the desired vials are retained. The kit can also include instructions for use, such as in printed or electronic format, such as digital format.

### Vl. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Expansion of Regulatory T Cells

The expression of CD9 was evaluated in resting CD4⁺ T cells isolated from cord blood or peripheral blood. Both T cells populations had a small percentage of CD4⁺CD9⁺ T cells. However, the T cells isolated from cord blood had a higher percentage of CD4⁺CD9⁺ T cells at 9.61% as compared to T cells isolated from peripheral blood at 6.40% (FIG. 1A). After expansion of the CD4⁺ T cells with IL-2, CD3/CD28 beads, and rapamycin, the Tregs were analyzed for CD9 expression. It was again found that Tregs expanded from cord blood had a higher percentage of CD4⁺CD9⁺ T cells (54.5%) as compared to those expanded from peripheral blood (25.6%) (FIG. 1B).

In addition, CD4 T cells were activated with CD3/CD28 beads to produce effector T cells for measurement of Treg suppressive ability. In a CFSE assay, the positive control showed almost 95% of the effector CD4 T cells proliferating. However, the addition of Tregs at a ratio of 1:5 or 1:1 of Tregs to CD4 T cells was able to suppress the proliferation of the CD4 T cells (FIG. 1C). The addition of cord blood Tregs decreased proliferation of activated CD4 T cells from about 94.8% to 33.9% at a 1:5 ratio and 13.1% at a 1:1 ratio. While the peripheral blood Tregs also showed suppressive function (from 80.2 to 63.8% at 1:5 and 35.3% at 1:1), their suppression level was lower than the cord blood Tregs. These data suggested that CD9⁺ Tregs are more immunosuppressive than CD9⁻ Tregs as the cord blood had a higher percentage of CD⁺ T cells.

To further evaluate the effect of CD9 expression on Treg suppressive function, the expanded Tregs were assayed for expression of several markers of suppressive activity. Comparison of CD9⁺ Tregs and CD9⁻ Tregs showed that CD9⁺ Tregs have a higher expression of markers of suppressive activity include CD39, CD73, CD15s, Neurophiline, and TIGIT (FIG. 2A).

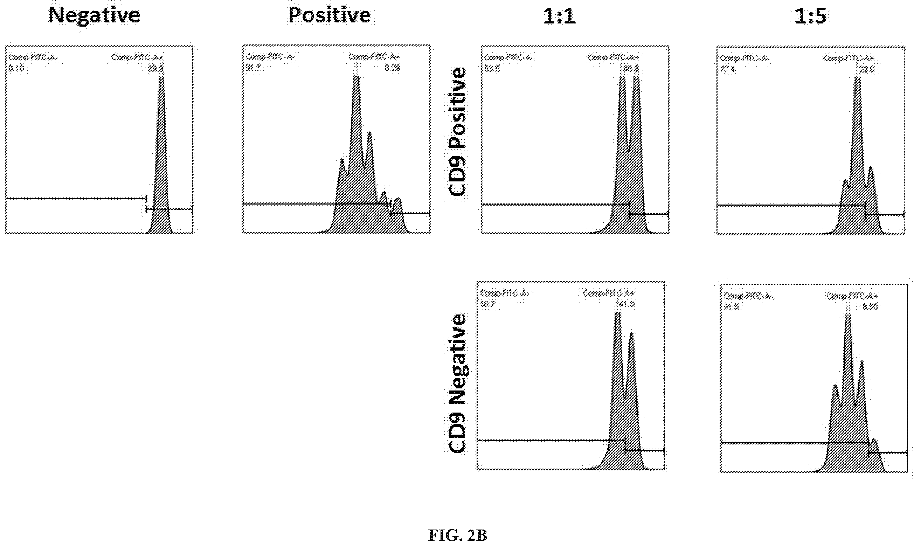

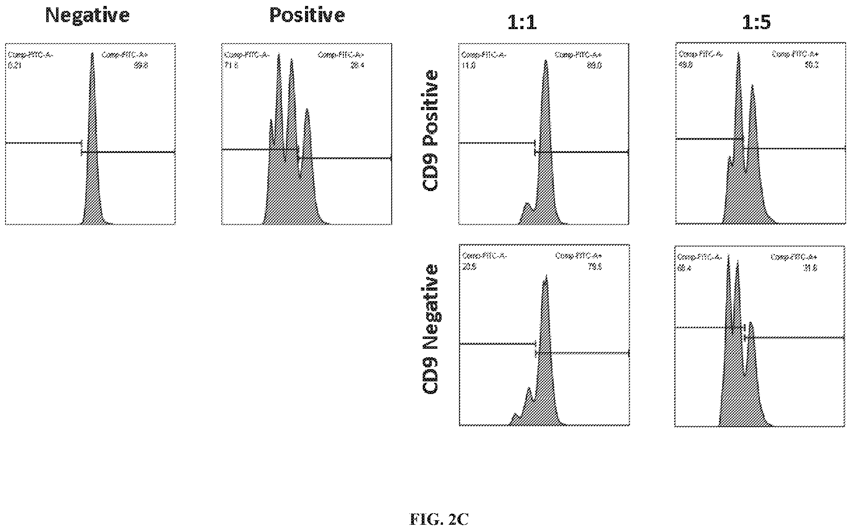

In addition, the CD9⁺ Tregs have higher suppressive activity when expanded from cord blood or peripheral blood as compared to CD9- Tregs (FIGS. 2B-2D). Specifically, the CD9⁺ peripheral blood Tregs suppressed proliferation of activated CD4 T cells at 46.5% at 1:1 and 22.6% at 1:5 while the CD9- peripheral blood Tregs only suppressed proliferation at 41.3% and 8.5%, respectively (FIG. 2B). The Tregs expanded cord blood showed an even more pronounced difference between the suppressive function of CD9⁺ of CD9⁻ Tregs. The CD9⁺ Tregs suppressed proliferation of activated CD4 T cells at 89.0% at 1:1 and 51.2% at 1:5, while the CD9⁻ Tregs suppressed proliferation at 79.5% and 31.6%, respectively (FIG. 2C). Thus, the CD9⁺ Tregs expanded from cord blood had almost two times the suppressive capacity as the CD9⁻ Tregs.

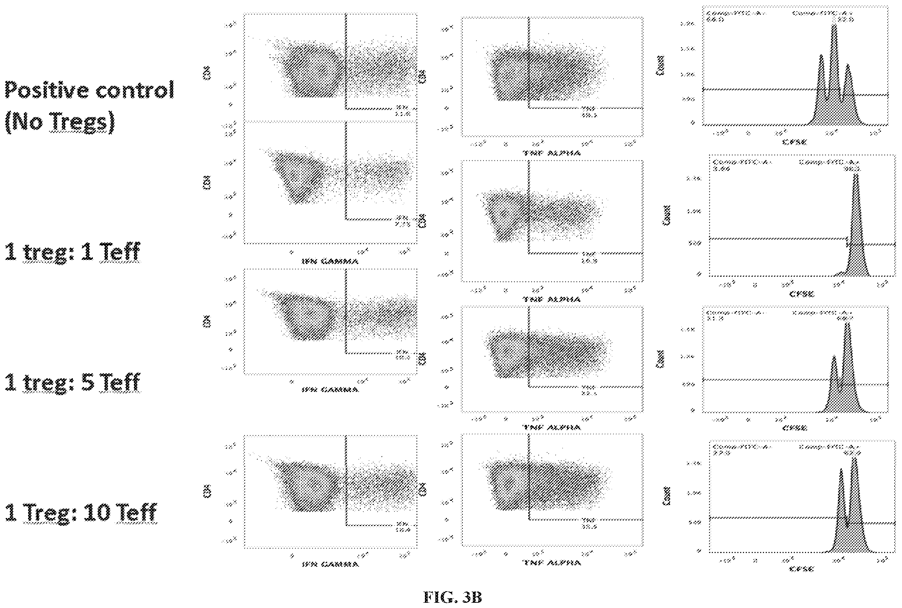

T cells were expanded by TCR ligation, IL-2, and rapamycin. Their suppressive effect on the proliferation of CFSE labeled CD4+ T cells and inflammatory cytokine production was measured. It was observed that Tregs expanded from fresh cord blood and expanded with TCR ligation and IL-2 had almost no suppressive function (FIG. 3A). However, Tregs expanded from fresh cord blood and expanded with TCR ligation, IL-2, and rapamycin were highly suppressive as observed by inhibition of the production of IFNγ and TNFα as well as decrease in the proliferation of effector T cells (FIG. 3B).

### Example 2 - Expansion of Regulatory B Cells

The suppressive function of regulatory B cells expanded by various methods was analyzed by measuring the levels of IFNγ, TNFα, and the proliferation of CFSE labeled CD4+ T cells. First, the B cells are isolated from a blood sample. Cord blood or peripheral blood is collected and Ficoll separation is performed. The cells are then stained for CD19, CD5, CD1d, and aqua live/dead dye. The cells are incubated with each of the monoclonal antibodies for 15-20 minutes in the dark at room temperature. Next, 2 mL of lysis buffer (BD Biosciences) is added and the cells are incubated for 3-5 at room temperature. T cells are then centrifuged at 1500 rpm for 5 min, the supernatant is discarded, 2 mL of PBS is added, and the cells centrifuged again at 1500 rpm for 5 min before discarding the supernatant. Sorting is then performed to select CD19⁺ or CD19⁺CD1d^{hi} CD5⁺ cells. Expansion may also be performed from whole B cells. The cells are counted and the appropriate cell culture plate or flask is selected.

For expansion, total B cells or B cell subsets were cultured in media with CD40L, IL-4, CpG as well as one signaling inhibitor. The signaling inhibitor was a FOXO1 inhibitor, an mTOR inhibitor, or a STAT6 inhibitor. After a few days, half of the media was replaced with fresh media containing IL-4 and a signaling inhibitor. In another 2-3 days, half of the media was again replaced with media containing IL-21, CD40L, and a signaling inhibitor. In addition, in some assays IL-33 was added to the media with IL-21. The combinations of IL-21+IL-33, IL-21+FOXO1 inhibitor, and IL-21+STAT6 inhibitor were all observed to produce highly suppressive Bregs (FIGS. 2A-2C).

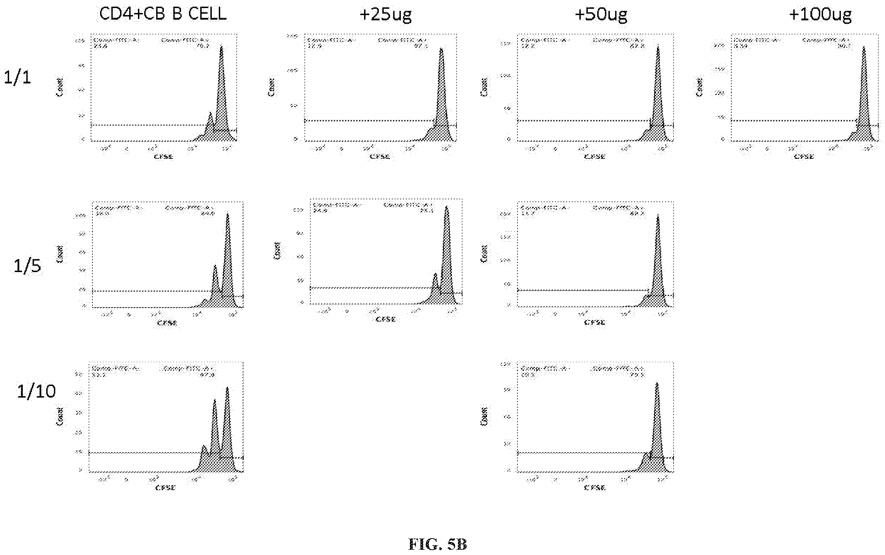

Next, the effect of miRNA-155 inhibitor on the activated Bregs was determined. The Bregs were incubated with miRNA-155 inhibitor for 1-2 hours. The Bregs were then cocultured with CD4 T cells that had been activated with CD3/CD28 beads to measure their suppressive activity. The Bregs were incubated with miRNA-155 inhibitor at a concentration of 25, 50, or 100 µg. As observed in FIG. 5B, the incubation with miRNA-155 inhibitor further increased the suppressive activity of the Bregs.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references, to the extent that they provide exemplary procedural or other details supplementary to those set forth herein, are specifically incorporated herein by reference.
Cohen et al., J Immunol. 175:5799-5808, 2005.
Current Protocols in Immunology, Ed Coligan et al, Wiley, 1994.
Czerkinsky et al., J. Immunol. Methods, 110:29-36, 1988.
EP 957359
Fast et al., Transfusion 44:282-5, 2004.
Fedorov et al., Sci. Transl. Medicine, 5(215), 2013.
He Y, et al. Journal of immunology research, 7, 2014.
Heemskerk et al. Hum Gene Ther. 19:496-510, 2008.
International Patent Publication No. WO2000/06588
International Patent Publication No. WO2000/06588
International Publication No. PCT/US95/01570
International Publication No. WO2000/06588
International Publication No. WO2005/035570
Janeway et al, Immunobiology: The Immune System in Health and Disease, 3rd Ed., Current Biology Publications, p. 433, 1997.
Johnson et al. Blood 114:535-46, 2009.
Lefranc et al., Dev. Comp. Immunol. 27:55, 2003.
Li, Nat Biotechnol. 23:349-354, 2005.
Olsson et al. J. Clin. Invest. 86:981-985, 1990.
Parkhurst et al., Clin Cancer Res. 15: 169-180, 2009.
PCT Patent Publication No. WO2001/083517
Taitano et al., The Journal of Immunology, 196, 201.6.
U.S. Patent 7,109,304
U.S. Patent No. 5,939,281
U.S. Patent No. 5,939,281
U.S. Patent No. 6,218,132
U.S. Patent No. 6,218,132
U.S. Patent No. 6,264,951
U.S. Patent No. 6,426,331
U.S. Patent No. 7,488,490
U.S. Patent No. 7,488,490
U.S. Patent Publication No. 2007/0078113
Varela-Rohena et al., Nat Med. 14: 1390-1395, 2008.
WO2014/055668
Wong et al., Cytotherapy, 4: 65-76, 2002.

### ASPECTS

1. An *in vitro* method for expanding CD9⁺ regulatory T cells (Tregs) comprising:
   (a) obtaining a population of CD4⁺ T cells;
   (b) culturing the population of CD4⁺ T cells under Treg expansion conditions, thereby producing expanded Tregs; and
   (c) selecting for CD9⁺ cells from the expanded Tregs, thereby obtaining a population of CD9⁺ Tregs.
2. The method of aspect 1, wherein the CD4⁺ T cells are further defined as CD4⁺CD25⁺ T cells.
3. The method of aspect 1 or 2, wherein selecting is further defined as positive selection.
4. The method of aspect 1 or 2, wherein selecting is further defined as negative selection.
5. The method of aspect 1 or 2, wherein selecting comprises sorting for CD9⁺ Tregs.
6. The method of aspect 5, wherein sorting is further defined as antibody bead selection, fluorescence associated cell sorting (FACS), or magnetic-activated cell sorting (MACS).
7. The method of aspect 1, wherein Treg expansion conditions comprise culturing the CD4⁺ T cells in the presence of TCR ligation, IL-2, and an mTOR inhibitor.
8. The method of aspect 7, wherein TCR ligation comprises an anti-CD3 antibody and an anti-CD28 antibody.
9. The method of aspect 7, wherein the mTOR inhibitor is rapamycin.
10. The method of any one of aspects 7-9, wherein the Treg expansion conditions further comprise culturing the CD4⁺ T cells in the presence of a tumor necrosis factor receptor 2 (TNFR2) agonist, all-trans retinoic acid (ATRA), adenosine receptor (A2AR), and/or an A2AR agonist.
11. The method of any one of aspects 1-9, wherein culturing is for 10-14 days.
12. The method of aspect 1, wherein obtaining the population of CD4⁺ T cells comprises isolating T cells from stem cells, bone marrow, peripheral blood, or cord blood.
13. The method of aspect 1, wherein obtaining the population of CD4⁺ T cells comprises isolating the T cells from pooled cord blood.
14. The method of aspect 12 or aspect 13, wherein isolating comprises performing antibody bead selection or fluorescence associated cell sorting (FACS).
15. The method of aspect 1, wherein the CD4⁺ T cells or Tregs are engineered to have decreased or essentially no expression of glucocorticoid receptor.
16. The method of aspect 15, wherein the CD4⁺ T cells or Tregs are engineered using one or more guide RNAs and a Cas9 enzyme.
17. The method of aspect 1, wherein the CD4⁺ T cells or Tregs are engineered to express a chimeric antigen receptor (CAR) and/or a T cell receptor (TCR).
18. The method of aspect 1, wherein the CD4⁺ T cells or Tregs are engineered to express a suicide gene.
19. The method of aspect 18, wherein the suicide gene is CD20, CD52, EGFRv3, or inducible caspase 9.
20. A population of CD9⁺ regulatory T cells produced according to the methods of any one of aspects 1-19.
21. A pharmaceutical composition comprising the population of CD9⁺ regulatory T cells of aspect 20 and a pharmaceutically acceptable carrier.
22. An *in vitro* method of expanding suppressive regulatory B cells (Bregs) comprising:
   (a) obtaining a population of B cells;
   (b) culturing the B cells in the presence of IL-4, CpG oligodeoxynucleotides (ODNs), and CD40 ligand (CD40L); and
   (c) further expanding the B cells in the presence of IL-21, CD40L, and at least one inhibitor selected from the group consisting of a FOXO1 inhibitor, a mTOR inhibitor, and a STAT6 inhibitor, thereby producing suppressive Bregs.
23. The method of aspect 22, wherein step (b) and/or step (c) further comprises one or more additional cytokines.
24. The method of aspect 23, wherein the additional cytokine is IL-33.
25. The method of aspect 22 or aspect 23, wherein the culturing of step (b) further comprises the presence of a FOXO1 inhibitor, a mTOR inhibitor, and/or a STAT6 inhibitor.
26. The method of aspect 22, further comprising washing the B cells prior to the expanding step.
27. The method of aspect 22, wherein CD40L is soluble CD40L (sCD40L).
28. The method of aspect 22, wherein the FOXO1 inhibitor is AS1842856 or AS1708727.
29. The method of aspect 22, wherein the FOXO1 inhibitor is AS1842856.
30. The method of aspect 22, wherein the mTOR inhibitor is torkinib, rapamycin, everolimus, temsirolimus, deforolimus, BGT226, SF1126, BEZ235, Gedatolisib, or SF1101.
31. The method of aspect 22, wherein the mTOR inhibitor is torkinib.
32. The method of aspect 22, wherein the STAT6 inhibitor is AS 1517499 or leflunomide.
33. The method of aspect 22, wherein the STAT6 inhibitor is AS1517499.
34. The method of aspect 22, further comprising contacting the Bregs with anti-miR-155.
35. The method of aspect 22, wherein obtaining the population of B cells comprises isolating B cells from a blood sample.
36. The method of aspect 35, wherein isolating comprises performing antibody bead selection or fluorescence associated cell sorting (FACS).
37. The method of aspect 35, wherein the blood sample is peripheral blood or cord blood.
38. The method of aspect 35, wherein the blood sample is cord blood (CB).
39. The method of aspect 38, wherein the cord blood is pooled from 2 or more individual cord blood units.
40. The method of aspect 38, wherein the cord blood is pooled from 3, 4, or 5 individual cord blood units.
41. The method of aspect 22, wherein the population of B cells are CB mononuclear cells (CBMCs).
42. The method of aspect 22, wherein the population of B cells are CD5⁺CD1d ^{hi} B cells.
43. The method of aspect 22, wherein the population of B cells are total B cells.
44. The method of aspect 22, wherein the Bregs have the capacity to suppress the proliferation of CD4⁺ T cells.
45. The method of aspect 22, wherein the Bregs are human Bregs.
46. The method of aspect 22, wherein the culturing is for 1 to 5 days.
47. The method of aspect 22, wherein the expanding is for 5 to 10 days.
48. A population of regulatory B cells produced according to the methods of any one of aspects 22-47.
49. A pharmaceutical composition comprising the population of regulatory B cells of aspect 48 and a pharmaceutically acceptable carrier.
50. A method of treating an immune disorder in a subject comprising administering a therapeutically effective amount of the suppressive Tregs of aspect 20 and/or the suppressive Bregs of aspect 48 to the subject.
51. The method of aspect 50, wherein the subject has been or is currently being administered a glucocorticoid therapy.
52. The method of aspect 50, wherein the immune disorder is inflammation, graft versus host disease, transplant rejection, or an autoimmune disorder.
53. The method of aspect 50, wherein the Tregs and/or Bregs are allogeneic.
54. The method of aspect 50, wherein the Tregs and/or Bregs are autologous.
55. The method of aspect 50, wherein the immune disorder is graft versus host disease (GVHD).
56. The method of aspect 55, wherein the GVHD is chronic GVHD (cGVHD).
57. The method of aspect 56, wherein the subject has been previously been administered a cord blood transplantation (CBT).
58. The method of aspect 57, wherein the Tregs and/or Bregs is administered concurrently with the CBT.
59. The method of aspect 57, wherein the Tregs and/or Bregs is administered prior to or after the CBT.
60. The method of aspect 50, wherein the immune disorder is transplant rejection, and wherein the transplant is an organ transplant, bone marrow or other cell transplant, composite tissue transplant, or a skin graft.
61. The method of aspect 50, wherein the immune disorder is multiple sclerosis, inflammatory bowel disease, rheumatoid arthritis, type I diabetes, systemic lupus erythrematosus, contact hypersensitivity, asthma or Sjogren's syndrome.
62. The method of aspect 50, wherein the subject is a human.
63. The method of aspect 50, further comprising administering at least a second therapeutic agent.
64. The method of aspect 63, wherein the at least a second therapeutic agent is a therapeutically effective amount of an immunomodulatory or an immunosuppressive agent.
65. The method of aspect 64, wherein the immunosuppressive agent is a calcineurin inhibitor, an mTOR inhibitor, an antibody, a chemotherapeutic agent irradiation, a chemokine, an interleukins or an inhibitor of a chemokine or an interleukin.
66. The method of aspect 63, wherein Tregs, Bregs, and/or the at least a second therapeutic agent are administered intravenously, intraperitoneally, intratracheally, intratumorally, intramuscularly, endoscopically, intralesionally, percutaneously, subcutaneously, regionally, or by direct injection or perfusion.

## Claims

1. An isolated CD4+ T cell engineered to have decreased expression of glucocorticoid receptor.

2. The isolated CD4+ T cell of claim 1, wherein the expression of glucocorticoid receptor is decreased by disrupting the gene encoding the glucocorticoid receptor.

3. The isolated CD4+ T cell of claim 2, wherein:
(a) the gene encoding the glucocorticoid receptor is knocked out;
(b) an insertion, missense or frameshift mutation is effected in the gene encoding the glucocorticoid receptor; or
(c) all or part of the gene encoding the glucocorticoid receptor is deleted.

4. The isolated CD4+ T cell of claim 2 or 3, wherein the gene encoding the glucocorticoid receptor is disrupted by a sequence-specific or targeted nuclease specifically designed to be targeted to the sequence of the gene or a portion thereof, optionally wherein the nuclease is:
(a) a DNA-binding targeted nuclease, optionally a zinc finger nuclease (ZFN) or a transcription activator-like effector nuclease (TALEN); or
(b) an RNA-guided nuclease, optionally a CRISPR-associated nuclease (Cas).

5. The isolated CD4+ T cell of claim 4, wherein the Cas is guided by a gRNA sequence of SEQ ID NO: 3 or 4.

6. The isolated CD4+ T cell of any one of the preceding claims, having essentially no expression of glucocorticoid receptor.

7. The isolated CD4+ T cell of any one of the preceding claims, engineered to express a chimeric antigen receptor (CAR) and/or a T cell receptor (TCR).

8. The isolated CD4+ T cell of any one of the preceding claims, engineered to express a suicide gene, optionally wherein the suicide gene is CD20, CD52, EGFRv3, or inducible caspase 9.

9. The isolated CD4+ T cell of any one of the preceding claims, wherein the CD4+ T cell is isolated from peripheral blood, cord blood, spleen, thymus, lymph nodes, or bone marrow.

10. The isolated CD4+ T cell of claim 9, wherein the CD4+ T cell is isolated by antibody bead selection or FACS.

11. The isolated CD4+ T cell of any one of the preceding claims, wherein the CD4+ T cell expresses CD25.

12. The CD4+ T cell of any one of the preceding claims, wherein the CD4+ T cell expresses CD9.

13. The isolated CD4+ T cell of any one of the preceding claims, wherein the CD4+ T cell has enhanced immunomodulation capacity, optionally wherein enhanced immunomodulation capacity is measured by CD4-positive or CD8-positive T cell suppression.

14. The CD4+ T cell of any one of the preceding claims, wherein the CD4+ T cell is a naive T (TN) cell, effector T cell (TEFF), memory T cell, tumor-infiltrating lymphocyte (TIL), immature T cell, mature T cell, helper T cell, cytotoxic T cell, mucosa-associated invariant T (MAIT) cells, regulatory T cell (Treg), helper T cell, alpha/beta T cell, or delta/gamma T cells.

15. The CD4+ T cell of any one of the preceding claims, wherein the CD4+ T cell is a human CD4+ T cell.
